# EUROPEAN PATENT SPECIFICATION

(11) **EP 0 610 265 B1**
(45) Date of publication and mention of the grant of the patent: **27.12.1996**
(21) Application number: 92921324.7
(22) Date of filing: 05.10.1992
(51) Int. Cl.: C07D 263/20, C07D 413/10, C07D 417/10, A61K 31/42, A61K 31/44, A61K 31/47, A61K 31/425

(54) **SUBSTITUTED ARYL- AND HETEROARYLPHENYLOXAZOLIDINONES USEFUL AS ANTIBACTERIAL AGENTS**
SUBSTITUIERTE ARYL- UND HETEROARYL-PHENYLOXAZOLIDINONE
ARYL- ET HETEROARYLPHENYLOXAZOLIDINONES SUBSTITUEES, UTILISEES COMME AGENTS ANTIBACTERIENS

(30) Priority: 01.11.1991 US 786107; 07.02.1992 US 831213
(43) Date of publication of application: 17.08.1994
(73) Proprietor: PHARMACIA & UPJOHN COMPANY, Kalamazoo, Michigan 49001 (US)
(72) Inventor: BARBACHYN, Michael, Robert, Kalamazoo, MI 49001 (US); BRICKNER, Steven, Joseph, Portage, MI 49002 (US)
(74) Representative: Perry, Robert Edward
(86) International application number: PCT/US92/08267
(87) International publication number: WO 93/09103

(56) References cited:
- EP-A- 0 127 902
- EP-A- 0 316 594
- EP-A- 0 352 781
- JOURNAL OF MEDICINAL CHEMISTRY vol. 33, no. 9, September 1990, WASHINGTON, USA, pages 2569-2578; WALTER A. GREGORY et al.: "Antibacterials. Synthesis and structure-activity studies of 3-aryl-2-oxooxazolidines".

## Description

### Field of the Invention

The present invention relates to novel substituted aryl- and heteroarylphenyloxazolidinones which are useful as anti-bacterial agents.

### Background of the Invention

The oxazolidinones are a class of orally-active, synthetic antibacterial agents and there are numerous references in the art disclosing a variety of oxazolidinone derivatives. For example, there are a number of references to 3-phenyl-2-oxazolidinone compounds having one or two substitutions on the phenyl ring. References disclosing a single substitution to the phenyl ring include U.S. Patent 4,948,801; 4,461,773; 4,340,606; 4,476,136; 4,250,318; 4,128,654, and Re 29,607. Additional references to 3-[(monosubstituted)phenyl]-2-oxazolidinones can be found in EP Publication 0 312 000, Gregory, et al., J. Med. Chem. 32:1673 (1989), Gregory, et al., J. Med. Chem. 33:2569 (1990), and Wang, et al., Tetrahedron 45:1323 (1989). Compounds of this type also include the antibacterial DuP721.

3-[(di- or fused-ring substituted)phenyl]-2-oxazolidinones are reported in U.S. Patents 4,977,173, 4,921,869, and 4,801,600; EP Publications 0 316 594, 0 184 170, and 0 127 902; and PCT Applications PCT/US89/03548 and PCT/US90/06220.

We have discovered 3-[(di- and tri-substituted)phenyl]-2-oxazolidinones which are effective as antibacterial agents. The compounds of the invention are characterized by oxazolidinones having an aryl or heteroaryl group at the *p*-position of the 3-phenyl ring and additional substitution(s) at the *m*-position of the phenyl ring with radicals having an electron-withdrawing effect. These compounds are surprisingly effective as antibacterial agents, since early work by Gregory, et al, J. Med. Chem. 33:2569 (1990), suggests compounds having such radicals in the *p*-position of the phenyl ring are less effective antibacterial agents.

Synthesis of 3-phenyl-2-oxazolidinones and derivatives thereof are well known in the art. However, due to the nature of the radicals, the substituted phenyls of the invention are difficult to synthesize. Thus, we also disclose a process by which the compounds of the invention may be synthesized.

### Information Disclosure

The following references disclose 3-phenyl-2-oxazolidinones having a single substitution on the phenyl ring:

U.S. Patent 4,948,801 discloses 3-[(aryl and heteroaryl)phenyl]-2-oxazolidinones having antibacterial activity.

U.S. Patent 4,476,136 discloses 3-[(*p*-arylalkyl, arylalkenyl, and arylacetylenic substituted)phenyl]-5-(aminomethyl)-2-oxazolidinones which have antibacterial activity.

U.S. Patent 4,461,773 discloses substituted 3-phenyl-5-(hydroxymethyl)-2-oxazolidinones which have antibacterial activity.

U.S. Patent 4,340,606 discloses substituted 3-[(p-alkylsulfonyl)phenyl]-5-(hydroxymethyl)- or (acyloxymethyl)-2-oxazolidinones having antibacterial activity in mammals.

U.S. Patent 4,250,318 discloses substituted 3-phenyl-5-(hydroxymethyl)-2-oxazolidinones having antidepressive utility.

U.S. Patent 4,128,654 discloses substituted 3-phenyl-5-(halomethyl)-2-oxazolidinones which are useful in controlling fungal and bacterial diseases of plants.

U.S. Reissue Patent 29,607 discloses substituted 3 phenyl-5-(hydroxymethyl)-2-oxazolidinones having antidepressive, tranquilizing and sedative utility.

Belgian Patent 892,270 discloses the 3-[(arylalkyl, arylalkenyl or arylacetylenic substituted)phenyl]-5-(aminomethyl)-2-oxazolidinones corresponding to U.S. Patent 4,476,136 listed above.

European Patent Publication 0 352 781 discloses aryl and heteroaryl substituted 3-phenyl-2- oxazolidinones corresponding to U.S. Patent 4,948,801 listed above.

European Patent Publication 0 312 000, as reported in Chemical Abstracts 89-116142/16, discloses phenylmethyl and pyridinylmethyl substituted 3-phenyl-2-oxazolidinones.

W. A. Gregory, et al., J. Med. Chem. 33:2569 (1990) and J. Med. Chem. 32:1673 (1989); C. J. Wang, et al., Tetrahedron 45:1323 (1989); and A. M. Slee, et al. Antimicrobial Agents and Chemotherapy 1791 (1987) are additional recent references disclosing 3-[(*p*-substituted) phenyl]-2-oxazolidinones.

The above references do not disclose the 3-[(di- or tri-substituted)phenyl]-2-oxazolidinones of the present invention.

The following references disclose 3-[(di-substituted)phenyl]- or 3-[(fused-ring substituted)phenyl]-2-oxazolidinones:

U.S. Patent 4,977,173 discloses 3-phenyl-2-oxazolidinones having a lactam at the *p*-position and fluorine at the *m*-position of the phenyl ring (Formula XIII). However, the 3-[(di- or tri-substituted)phenyl]-2-oxazolidinones of the present invention have an aromatic ring at the *p*-position.

U.S. Patents 4,921,869 and 4,801,600 disclose 6'-indolinyl- or alkanoneoxazolidinones (where the indolinyl nitrogen is meta to the oxazolidinone nitrogen).

U.S. Patent 4,705,799 discloses substituted aminomethyloxooxazolidinyl benzene derivatives including sulfides, sulfoxides, sulfones and sulfonamides which possess antibacterial activity. However, compounds of the present invention have an aryl or heteroaryl at the p-position of the phenyl ring.

European Patent Publication 0 316 594 discloses substituted 3-(styryl)-2-oxazolidinones corresponding to U.S. Patent 4,977,173 listed above.

European Patent Publications 0 184 170 and 0 127 902 correspond to U.S. Patent 4,705,799, discussed above.

PCT/US89/03548 and PCT/US90/06220 disclose 3-[(fused-ring substituted)phenyl]-2-oxazolidinones which are useful as antibacterial agents.

The above references do not disclose the 3-[(di- or tri-substituted)phenyl]-2-oxazolidinones of the present invention.

### SUMMARY OF THE INVENTION

Disclosed are substituted aryl- and heteroaryl-phenyl oxazolidinones of Formula (XII) 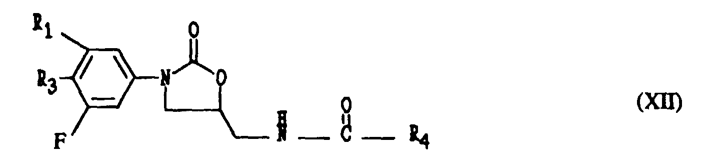 where
(I) R₁ is
   (a) -H,
   (b) -F,
   (c) -Cl,
   (d) -CF₃, and
   (e) -OCH₃;
(II) R₃ is selected from the group consisting of
   (a) phenyl,
   (b) pyridyl,
   (c) pyrazinyl, (d) pyridazinyl, (e) pyrimidinyl,
   (f) 1,2,3-, (g) 1,2,4-, (h) 1,2,5-triazinyl,
   (i) quinolinyl, (j) isoquinolinyl,
   (k) quinoxalinyl, (l) quinazolinyl, (m) phthalazinyl, (n) cinnolinyl,
   (o) naphthyridinyl,
   (p) indolyl having nitrogen optionally substituted with R₅₋₁ where R₅₋₁ is
      -H,
      C₁-C₄ alkyl optionally substituted with one or more halogens,
      C₃-C₆ cycloalkyl, or
      -C(O)R₅₋₂ where R₅₋₂ is
         -H,
         C₁-C₄ alkyl optionally substituted with one or more halogens, or
         phenyl optionally substituted with one or more halogens,
   (q) pyrrolopyridinyl having the saturated nitrogen substituted with R₅₋₁ where R₅₋₁ is as defined above, (r) furanopyridinyl, (s) thienopyridinyl,
   (t) benzothiazolyl, (u) benzoxazolyl,
   (v) imidazolyl having the saturated nitrogen substituted with R₅₋₁ where R₅₋₁ is as defined above,
   (w) pyrazolyl having the saturated nitrogen substituted with R₅₋₁ where R₅₋₁ is as defined above,
   (x) thiazolyl, (y) isothiazolyl,
   (z) oxazolyl, (aa) isoxazolyl,
   (bb) pyrroyl having nitrogen substituted with R₅₋₁ where R₅₋₁ is as defined above,
   (cc) furanyl, (dd) thiophenyl,
      wherein substitutents (a)-(dd) are optionally substituted with X and Y,
   (ee) 1,2,3-, (ff) 1,2,4-triazolyl having the saturated nitrogen substituted with R₅₋₁ where R₅₋₁ is as defined above,
   wherein substituents (ee) and (ff) are optionally substituted with X;
(III) each occurrence of Y is independently selected from
   (a) -H,
   (b) -Fl, (c) -Cl, (d) -Br, (e) -I,
   (f) -R₃₋₁, (g) -OR₃₋₁, where R₃₋₁ is H or C₁-C₄ alkyl, or
   (h) -NO₂;
(IV) each occurrence of X is independently selected from
   (a) -H,
   (b) C₁-C₈ alkyl optionally substituted with
      one or more halogens.
      -OH,
      =O other than at alpha position,
      -S(O)ₙR₃₋₂ where R₃₋₂ is C₁-C₄ alkyl or C₃-C₈ cycloalkyl, or
      -NR₃₋₃R₃₋₄ where R₃₋₃ and R₃₋₄ are the same or different and are -H,
      C₁-C₈ alkyl, C₃-C₈ cycloalkyl, -(CH₂)ₜCHOR₃₋₅, -(CH₂)ₜNR₃₋₆R₃₋₇, or taken together are -(CH₂)O(CH₂)-,
      -(CH₂)ₜCH(CO)R₃₋₈, or -(CH₂)N(R₃₋₈)(CH₂)₂- where
      R₃₋₅ is -H or C₁-C₄ alkyl, or
      R₃₋₆ and R₃₋₇ are the same or different and are -H, C₁-C₄ alkyl or taken together are -(CH₂)ᵣ-,
   (c) C₂-C₅ alkenyl,
   (d) C₃-C₈ cycloalkyl,
   (e) -OR₃₋₃ where R₃₋₃ is as defined above,
   (f) -CN,
   (g) -S-(O)ₙ-R₃₋₈ where R₃₋₈ is
      C₁-C₄ alkyl optionally substituted with
         one or more halogens,
         -OH,
         -CN,
         -NR₃₋₃R₃₋₄ where R₃₋₃ and R₃₋₄ are as defined above, or
         -CO₂R₃₋₅ where R₃₋₅ is as defined above.
      C₂-C₄ alkenyl,
      -NR₃₋₉R₃₋₁₀ where R₃₋₉ is -H, C₁-C₄ alkyl, or C₃-C₈ cycloalkyl and R₃₋₁₀ is -H, C₁-C₄ alkyl, C₁-C₄ alkenyl, C₃-C₄ cycloalkyl, -OR₃₋₅, or -NR₃₋₆R₃₋₇ where R₃₋₅, R₃₋₆, and R₃₋₇ are as defined above,
      -N₃,
      -NHC(O)R₃₋₁₁ where R₃₋₁₁ is C₁-C₄ alkyl optionally substituted with one or more halogens,
   (h) -S(O)₂-N=S(O)ₚR₃₋₁₄R₃₋₁₅ where R₃₋₁₄ and R₃₋₁₅ are the same or different and are C₁-C₂ alkyl, or taken together are -(CH₂)_{q}-,
   (i) -S-C(O)-R₃₋₁₁ where R₃₋₁₁ is as defined above,
   (j) tetrazoly,
   (k) -NR₃₋₃R₃₋₄ where R₃₋₃ and R₃₋₄ are as defined above,
   (l) -N(R₃₋₃)COR₃₋₁₁ where R₃₋₃ and R₃₋₁₁ are as defined above,
   (m) -N(R₃₋₃)S(O)ₙR₃₋₁₁ where R₃₋₃ and R₃₋₁₁ are as defined above,
   (n) -CONR₃₋₃R₃₋₄ where R₃₋₃ and R₃₋₄ are as defined above,
   (o) -C(O)R₃₋₁₆ where R₃₋₁₆ is
      -H,
      C₁-C₈ alkyl optionally substituted with one or more halogens,
      C₁-C₄ alkyl optionally substituted with
         -OR₃₋₅,
         -OC(O)R₃₋₅,
         -NR₃₋₃R₃₋₄,
         -S(O)ₙR₃₋₁₇,
      C₃-C₈ cycloalkyl, or
      C₂-C₅ alkenyl optionally substituted with -CHO or CO₂R₃₋₅, where R₃₋₃, R₃₋₄, and R₃₋₅ are as defined above and R₃₋₁₇ is C₁-C₄ alkyl or C₃-C₈ cycloalkyl,
   (p) -C(=NR₃₋₁₈)R₃₋₁₆ where R₃₋₁₆ is as defined above and R₃₋₁₈ is -NR₃₋₃R₃₋₄.
      -OR₃₋3, or -NHC(O)R₃₋₃ where R₃₋₃ and R₃₋₄ are as defined above,
   (g) -CR₃₋₁₆(OR₃₋₁₉)OR₃₋₂₀ where R₃₋₁₆ is as defined above and R₃₋₁₉ and R₃₋₂₀ are the same or different and are C₁-C₄ alkyl, or taken together are -(CH₂)ₘ-, 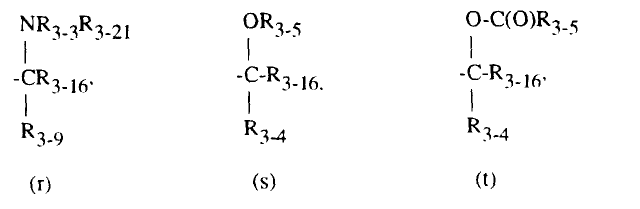 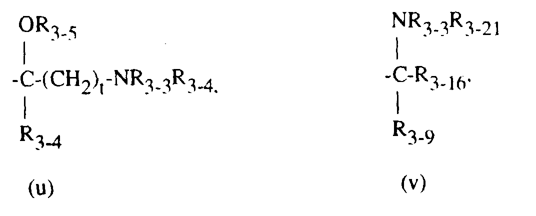 where R₃₋₃, R₃₋₄, R₃₋₅, R₃₋₉, and R₃₋₁₆ are as defined above and R₃₋₂₁ is R₃₋₄ or -NR₃₋₄R₃₋₅ where R₃₋₄ and R₃₋₅ are as defined above;
      m is 2 or 3;
      n is 0, 1, or 2;
      p is 0 or 1;
      q is 3, 4, or 5;
      t is 1, 2, or 3;
      (V) R₄ is selected from the group consisting of
         (a) -H,
         (b) C₁-C₁₂ alkyl optionally substituted with 1-3 Cl,
         (c) C₃-C₁₂ cycloalkyl,
         (d) C₅-C₁₂ alkenyl containing one double bond,
         (e) phenyl optionally substituted with 1-3 -OH, -OCH₃, -OC₂H₅, -NO₂, -F, -Cl, -Br, -COOH and -SO₃H, -N(R₄₋₁)(R₄₋₂) where R₄₋₁ and R₄₋₂ are the same or different and are -H and C₁-C₅ alkyl,
         (f) furanyl,
         (g) tetrahydrofuranyl.
         (h) 2-thiophene,
         (i) pyrrolidinyl,
         (j) pyridinyl,
         (k) -O-R₄₋₃ where R₄₋₃ is C₁-C₄ alkyl,
         (l) -NH₂,
         (m) -NHR₄₋₄ where R₄₋₄ is C₁-C₃ alkyl or -φ,
         (n) -NR₄₋₄R₄₋₅ where R₄₋₄ is as defined above and R₄₋₅ is C₁-C₃ alkyl, or taken together with the attached nitrogen atom to form a saturated mono-nitrogen C₅-C₇ heterocyclic ring including -O- (morpholine),
         (o) -CH₂-OH,
         (p) -CH₂-OR₄₋₆ where R₄₋₆ is C₁-C₄ alkyl or -CO-R₄₋₇ where R₄₋₇ is C₁-C₄ alkyl or -φ;
and pharmaceutically acceptable salts thereof.

More particularly, the invention discloses compounds of formula (XII) where R₃ is a pyridyl or phenyl ring which are optionally substituted with -H, C₁-C₄ alkyl, or -NR₃₋₃R₃₋₄.

Most particularly, disclosed are the compounds (±)-5-(acetamidomethyl)-3-[4-(3-pyridyl)-3,5-difluorophenyl]-2-oxazolidinone and (±)-5-(acetamidomethyl)-3-[4-(4-pyridyl)-3,5-difluorophenyl] -2-oxazolidinone.

Another aspect of the invention discloses a process for making a compound of formula (XII) comprising:
(a) converting a substituted aniline to a stabase derivative,
(b) treating the stabase derivative to form an aryl- or heteroaryl-substituted aniline, and
(c) converting the aryl- or heteroaryl-substituted aniline to a aryl- or heteroaryl-substituted phenyloxazolidinone.

More particularly, this aspect of the invention discloses a process for making a compound of formula (XII) wherein step (b) comprises treatment of the stabase derivative with an appropriate alkyl- or aryllithium to form a lithiated derivative, transmetallation with an appropriate electrophilic metal species, addition of an appropriate aryl- or heteroaryl halide or sulfonate precursor in the presence of an appropriate metal catalyst to form a protected aryl- or heteroarylaniline, and removal of the stabase protecting group with aqueous mineral acid.

Most particularly, this aspect of the invention discloses a process for making a compound of formula (XII) wherein step (b) is carried out in a one-pot reaction sequence involving deprotonation of the stabase derivative with *n*-butyllithium in tetrahydrofuran to form a lithiated derivative, transmetallation with zinc chloride, addition of an aryl- or heteroarylbromide, iodide, triflate, or fluorosulfonate in the presence of tetrakis(triphenylphosphine)palladium catalyst to form a protected aryl- or heteroaryl- aniline, and deprotection with aqueous hydrochloric acid.

Still another aspect of the invention discloses a process for making an oxazolidinone iodide comprising reacting a carbobenzyloxy allyl compound in the presence of an excess of pyridine and iodine, said excess being of equal amounts in the range of 2-20 molar equivalents.

More particularly, this aspect of the invention discloses a process for making an oxazolidinone iodide comprising reacting a carbobenzyloxy allyl compound in the presence of an excess of pyridine and iodine, wherein said excess is in the range of 5-15 molar equivalents.

Most particularly, this aspect of the invention discloses a process for making an oxazolidinone iodide comprising reacting a carbobenzyloxy allyl compound in the presence of an excess of pyridine and iodine, wherein said excess is in the range of 8-10 molar equivalents.

### DETAILED DESCRIPTION OF THE INVENTION

It is preferred that R¹ is -F or -CF₃; it is most preferred that R₁ is -F.

It is preferred that R₃ is phenyl or pyridyl; it is most preferred that R₃ is 3-pyridyl or 4-pyridyl.

It is preferred that X is -H, C₁-C₄ alkyl, or -NR₃₋₃R₃₋₄; it is most preferred that X is -H.

It is preferred that Y is -H or C₁-C₄ alkyl; it is most preferred that Y is -H.

It is preferred that R₄ is an acyl group optionally substituted with C₁-C₅ alkyl optionally substituted with 1-3 halogens, C₃-C₅ cycloalkyl, -NR₃₋₃R₃₋₄, and -OR₃₋₃; it is most preferred that R₄ is -CH₃.

It is preferred that R₅ is C₁₋C₄ alkyl.

The structures of the aryl and heteroaryl groups which comprise R₃ (I-XII) are shown in CHART C. Structures (o) (naphthyridinyl), (q) (pyrrolopyridinyl), (r) (furanopyridinyl), and (s) (thienopyridinyl) show the nitrogen-containing heteroaryl abbreviated as Z, where Z is an unsaturated 4-atom linker having one nitrogen and three carbons. In this way, each of the four possible positions for the heteroaryl nitrogen are encompassed by structures (o), (q), (r), and (s).

The aryl- and heteroaryl-substituted phenyloxazolidinones (XII) of the present invention are useful as antibacterial agents in treating infections in mammals caused by gram-positive and anaerobic infection. It is preferred to treat humans and warm-blooded mammals such as cattle, horses, sheep, hogs, dogs, cats, etc., with the aryl- and heteroaryl-substituted phenyl oxazolidinones (XII) of the present invention.

The aryl- and heteroaryl-substituted phenyloxazolidinones (XII) of the present invention are also useful in treating patients infected with one or more *Mycobacterium spp.* Of particular interest, the aryl- and heteroaryl-substituted phenyloxazolidinones (XII) of the invention are useful in treating patients infected with *M. tuberculosis* and M. *avium.*

The aryl- and heteroaryl-substituted phenyloxazolidinones (XII) of the invention can be administered in a manner and in dosage forms similar to those of the known phenyloxazolidinones described above. For example, administration can be either parenteral (IV, IM, SQ) or oral. The daily dose is about 3 to about 30 mg/kg. This dose can preferably be given in divided doses and administered 2-4 times daily. The preferred route of administration as well as the particular dosage form for either the parenteral or oral route depends on the particular facts of the situation including the nature of the infection and condition of the patient. The usual pharmaceutical dosage forms appropriate for parenteral (solution, suspension in oil) and oral (tablet, capsule, syrup, suspension, etc) administration are known to those skilled in the art and there is nothing unusual about using those dosage forms with the aryl- and heteroaryl-substituted phenyloxazolidinones (XII). The exact dosage of the aryl- and heteroaryl-substituted phenyloxazolidinones (XII) to be administered, the frequency of administration, route of administration, and the dosage form will vary depending on a number of factors known to those skilled in the art including the age, weight, sex, general physical condition of the patient, the nature of the infection (particular microorganism involved, its virulence, the extent of the infection) other medical problems of the patient, etc., as is well known to the physician treating infectious diseases.

The aryl- and heteroaryl-substituted phenyloxazolidinones (XII) can be used either alone or in conjunction with other antibacterial agents as is known to those skilled in the art. Further, the aryl- and heteroaryl-substituted phenyloxazolidinones (XII) can be used in conjunction with non-antibacterial agents as is known to those skilled in the art.

Suitable pharmaceutical salts include the acid addition salts when a basic group is present, such as occurs with the preferred pyridyl group. The acid addition salts including those made from mineral acids, e.g., hydrochloric, hydrobromic, sulfuric, phosphoric, etc., organic sulfonic acids, e.g., methanesulfonic, organic carboxylic acids, e.g., amino, and carbohydrate acids, e.g., gluconic, galacturonic, etc. It is also appreciated by those skilled in the art that the appropriate N-oxides of R₃ heteroaryls and tertiary amino substituted aryls are included within the scope of the aryl- and heteroaryl-substituted phenyloxazolidinones (XII) of the invention

The pharmaceutically active aryl- and heteroaryl-substituted phenyloxazolidinones (XII) of this invention are prepared as described briefly here and in more detail in the examples which follow. CHART A describes the synthesis of the aryl- and heteroaryl-substituted aniline (VI) compounds. Here and in other Charts, R₂ represents F.

These aniline compounds (VI) are then subsequently reacted following procedures known, or readily acquired by one skilled in the art. These subsequent procedures parallel those described in U.S. Patents 4,705,799, PCT/US90/06220, PCT/US89/03548, and W. A. Gregory, et al., J. Med. Chem., 32:1673 (1989), all of which are incorporated herein by reference. The Cardillo-Ohno reaction is discussed in Tetrahedron 43:2505 (1979) and Tetrahedron Lett. 28:3123 (1987), both of which are also incorporated herein by reference.

CHART A demonstrates a method of preparation of the aryl- and heteroaryl-substituted anilines of the invention. The starting point is a mono- or disubstituted aniline (I). These materials are readily available from a number of commercial vendors; one such vendor is Aldrich Chemical Co., Milwaukee, Wl. Alternatively, the anilines (I) are known in the chemical literature and may be readily prepared by one skilled in the art. The substituted aniline (I) is treated with n-butyllithium and 1,2-bis(chlorodimethylsilyl)ethane to form the stabase (SB) derivative (II). The SB derivative (II) is converted, in a one-pot reaction sequence, to the crude aryl- and heteroaryl-substituted aniline (VI). This sequence involves slow addition of n-butyllithium to (II), resulting in the aryllithium (III). The aryllithium (III) is transmetallated with anhydrous zinc chloride in tetrahydrofuran (THF) to give an organo zinc derivative (IV). Alternatively, the transmetallation can be carried out with trimethylborate to give the corresponding boric acid, or with tributyltin chloride to give the corresponding stannane. These species react in a manner analogous to that of the organozinc derivative (IV). Addition of the appropriate aryl or heteroaryl iodide, bromide, trifluoromethane sulfonate (triflate), or fluorosulfonate and palladium catalyst, preferably, tetrakis(triphenylphosphine)palladium, followed by warming to reflux temperature gives the coupled product (V). The reaction is quenched with aqueous mineral acid, preferably hydrochloric, to give the aryl- or heteroaryl-substituted aniline (VI). The product (VI) may then be further purified following chromatographic techniques well known in the art. Altematively, the chromatographic purification may be delayed until after the carbobenzoxy group is appended.

The remaining synthetic steps which lead to the aryl- and heteroaryl-substituted phenyl oxazolidinone (XII) of the invention are outlined in CHART B and closely parallel the procedure found in that discussed in U.S. Patent 4,705,799, PCT/US90/06220, PCT/US89/03548, and W. A. Gregory, et al., J. Med. Chem., 32:1673 (1989). Briefly, the aniline (VI) is converted to the carbobenzoxy derivative (VII) in the presence of base and THF. Alkylation of (VII) with allyl halide, preferably bromide, gives the allylated product (VIII). This intermediate (VIII) is subjected to a modified Cardillo-Ohno iodocyclocarbamation reaction wherein an excess of pyridine in combination with the iodine, is added to facilitate formation of the oxazolidinone iodide (IX). The iodocyclocarbamation reaction as disclosed by Cardillo-Ohno requires 2-3 equivalents of iodine (I₂), whereas when synthesizing the compounds of the invention an amount of pyridine and iodine is added which is in the range of 2-20 molar equivalents of each compound. Preferably, 5-15 equivalents should be employed, most preferred is 8-10 equivalents. In addition, we have found that this process has utility in reactions where benzyl iodide is formed and the product so formed competes in the reaction for the substrate and/or product to form unwanted benzylated products. This process, then, is useful to improve yields because it is believed that the excess pyridine traps the benzyliodide to inhibit this competitive reaction. In the synthesis of the compounds of the invention an excessive of pyridine is necessary; when pyridine is omitted from the reaction mixture essentially none of the compounds of the invention are recovered.

After the formation of the iodide (IX) purification may be accomplished following chromatography procedures known in the art. However, this is not necessary as the iodide (IX) may be directly converted to the corresponding azide (X) by treatment with sodium azide in the presence of DMF. Reduction of (X) in the presence of hydrogen, methanol or ethyl acetate, and palladium catalyst affords the amine (XI). Acetylation of the amine (XI) provides the aryl-and heteroaryl-substituted phenyloxazolidinones (XII) of the invention.

The aryl- and heteroaryl-substituted phenyloxazolidinones (XII) of the invention contain at least one chiral center. It is apparent to one skilled in the art that when one chiral center is present, the compound can exist as one of two possible optical isomers [(R) and (S) enantiomers] or a racemic mixture of both. Both individual (R) and (S) enantiomers, as well as mixtures thereof, are within the scope of aryl- and heteroaryl-substituted phenyloxazolidinones (XII) of the invention. In the event a second chiral center is present in the aryl- and heteroaryl-substituted phenyloxazolidinones (XII) of the invention, the resultant diastereomers, in racemic and enantiomerically enriched forms, are also within the scope of the compounds (XII) of the invention.

The enantiomer which is pharmacologically active is the enantiomer with the "S" configuration. The racemic mixture is useful in the same way and for the same purpose as the pure S-enantiomer; the difference is that twice as much racemic material must be used to produce the same effect as the pure S-enantiomer. If desired, the mixture of enantiomers is resolved by means known to those skilled in the art. It is preferable to resolve the racemic mixture at the stage of the amino compounds (XI) using methods known to those skilled in the art, see for example, Optical Resolution Procedures for Chemical Compounds, Vol 1,: Amines and Related Compounds, Paul Newman, Optical Resolution Information Center, Manhattan College, Riverdale, NY, 10471, 1978. For example, treatment of the d,l-amino mixture (XI) with an optically active acid such as (+)-tartaric acid or alternatively with (-)-tartaric acid, would yield a mixture of diastereomeric salts, which can be separated most conveniently by fractional crystallization to give a salt containing only one enantiomer of the racemic mixture. Other suitable optically active acids include, (-) dibenzoyltartaric acid. (+)-camphoric acid, (+)- and (-)-malic acid and (+)-camphor-10-sulfonic acid. By reacting the diastereomeric salt with a base one obtains the enantiomer as the free amino compound (XI). These optically pure compounds are then used in the same way as the racemic mixture.

Charts D and E depict alternative and preferred routes to enantiomerically enriched substituted aryl- and heteroarylphenyloxazolidinones of formula XII which are the subject of this invention. It will be apparent to those skilled in the art that these are merely representative examples, and that slight modifications of the provided synthetic protocols will allow for the preparation of further enantiomerically enriched examples of the oxazolidinones of the invention.

The reaction of an isocyanate with racemic and enantiomerically enriched glycidol derivatives to give oxazolidinones is a known and facile process. (See e.g., J. E. Herweh, W. J. Kauffman, Tetrahedron Letter., 809 (1971); W. A. Gregory, et al., J. Med. Chem., 32:1673 (1989); W. A. Gregory, et al., J. Med. Chem. 33:2569 (1990); C. H. Park, et al., J. Med. Chem., 35:1156 (1992); W. A. Gregory, U.S. Patent 4,705,799 (1987)). As shown in Chart D, an isocyanate of structure (1) can be reacted with commercially available (R)-glycidyl butyrate (see, e.g., W. E. Ladner, G. M. Whitesides; J. Am. Chem. Soc., 106:7250 (1984), available from Aldrich Chemical Company, Inc.) in the presence of catalytic lithium bromide and tributylphosphine oxide and in a suitable solvent such as xylene and at a suitable temperature (e.g. reflux) to provide the oxazolidinone intermediate (2). The butyryl group is then removed by reaction with an alkoxide, preferably sodium methoxide in methanol, to furnish the key hydroxymethyloxazolidinone intermediate (3). The most preferred route to the alcohol (3) involves the deprotonation of an appropriate CBz-protected aniline of structure (4), readily prepared by standard Schotten-Baumann conditions or other variations known to one skilled in the art, with a suitable base such as *n*-butyllithium in a suitable solvent such as tetrahydrofuran and at a suitable temperature such as -78° to -60°C. Addition of the (*R*)-glycidyl butyrate, followed by warming to ambient temperature, then directly affords the hydroxymethyloxazolidinone (3), identical in all respects with material prepared via the isocyanate sequence. Compound (3) is then converted to the corresponding methanesulfonate (mesylate) or *p*-toluenesulfonate (tosylate) derivative (5) or the like, by the action of methanesulfonyl chloride/pyridine or methanesulfonyl chloride/triethylamine/dichloromethane or *p*-toluenesulfonyl chloride/pyridine. The resultant sulfonate is then reacted with an azide source, for example sodium or potassium azide, in a dipolar aprotic solvent such as *N,N*-dimethylformamide (DMF) or 1-methyl-2-pyrrolidinone optionally in the presence of a catalyst such as 18-crown-6 at a temperature of 50° to 90°C to afford the azide (6). The azide (6) is then reduced by hydrogenation with palladium on carbon or a platinum-based catalyst in an appropriate solvent such as ethyl acetate or methanol (or combina-tions thereof) to give the aminomethyloxazolidinone (7). Alternatively, the azide may be reduced by treatment with a trivalent phosphorus compound such as triphenylphosphine in the presence of water and in a suitable solvent such as tetrahydrofuran (THF). Compound (7) is then acylated by reactions known to those skilled in the art to give the intermediates of structure (8). Compound (8) is then iodinated with iodine monochloride in acetic acid or acetic acid/trifluoroacetic acid (see. e.g., W. A. Gregory, U.S. Patent 4,705,799 (1987)) at a temperature from 0° to 70°C or with iodine and silver trifluoroacetate (see e.g., W. A. Gregory, *et al.,* J. Med. Chem. 33:2569 (1990); and C. H. Park, et al., J. Med. Chem., 35:1156 (1992)) to furnish the enantiomerically enriched substituted iodophenyloxazolidinone intermediate (9). Alternatively, (8) can be brominated with *N*-bromosuccinimide to give the corresponding bromophenyloxazolidinone of structure 9.

Further elaboration of the intermediates of formula (9) to make the enantiomerically enriched substituted aryl- and heteroarylphenyloxazolidinones of formula XII which are the subject of this invention is outlined in Chart E. Compound (9) is reacted with the desired aryl- or heteroaryl-substituted metal of formula R³M (M = trialkyltin, boronic acid or ester, or halozinc) in the presence of a suitable palladium catalyst such as tetrakis(triphenylphosphine)palladium or bis(triphenylphosphine)palladium chloride in a suitable solvent such as DMF or 1,4-dioxane at a suitable temperature (typically 70°-100°C) to afford the coupled aryl- or heteroarylphenyloxa-zolidinone products of structure XII. Altematively, the iodo- or bromophenyloxazolidinone of formula (9) is converted to the corresponding trimethyltin derivative (11) by treating it with hexa-methylditin in the presence of a suitable palladium catalyst such as tetrakis(triphenylphosphine)-palladium or bis(triphenylphosphine)palladium chloride in a suitable solvent such as DMF or 1,4-dioxane at a suitable temperature (typically 70° to 100°C). Intermediate (11) is then treated with the desired aryl or heteroaryl halide of formula R³X (X = Br or I) in the presence of a suitable palladium catalyst such as tetrakis-(triphenylphosphine)palladium or bis(triphenyl-phosphine)palladium chloride in a suitable solvent such as DMF or 1,4-dioxane at a suitable temperature (typically 70°-100°C) to afford the enantiomerically enriched aryl- or heteroaryl-phenyloxazolidinone products of structure XII which are the subject of this invention. This latter route (proceeding through 11) is exemplified in the supplemental experimental section by a racemic Example 55.

The definitions and explanations below are for the terms as used throughout this entire document including both the specification and the claims.

### Conventions

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-C(=Z₁)H= Groups Rᵢ and Rⱼ would represent monovalent variable substituents if attached to the formula CH₃-CH₂-C(Rᵢ)(Rⱼ)H₂. When chemical formulas are drawn in a linear fashion, such as those above, variable substituents contained in parentheses are bonded to the atom immediately to the left of the variable substituent enclosed in parenthesis. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom.

Chemical formulas or portions thereof drawn in a linear fashion represent atoms in a linear chain. The symbol "-" in general represents a bond between two atoms in the chain. Thus, CH₃-O-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N*=C(CH₃)-CH=CCl-CH=C*H with the convention that the atoms marked with an asterisk (*) are bonded to each other resulting in the formation of a ring. Likewise, the cyclic molecular fragment, 4-(ethyl)-1-piperazinyl can be represented by -N*-(CH₂)₂-N(C₂H₅)-CH₂-C*H₂.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In chemical structural formulas depicting such compounds, a substituent (X₁) which is "below" another substituent (X₂) will be identified as being in the alpha (α) configuration and is identified by a broken, dashed or dotted line attachment to the carbon atom, i.e., by the symbol "- - -" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as -C(=Rᵢ)- might be bivalent and be defined as oxo or keto (thus forming a carbonyl group (-CO-)) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(=R₆)- is defined to consist of two monovalent variable substituents, the two monovalent variable substituents are α-R₆₋₁:β-R₆₋₂, ... α-R₆₋₉:β-R₆₋₁₀, etc, giving -C(α-R₆₋₁)(β-R₆₋₂)-, ... -C(α-R₆₋₉)(β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..." means a lactone in which the carbonyl is bonded to C₂. However, when designated "... Rⱼ and Rᵢ are taken together to form -CO-O-CH₂-CH₂-the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-0-CO- where n is zero, one or two. By the second method, the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

### Definitions

All temperatures are in degrees Centigrade.

TLC refers to thin-layer chromatography.

Brine refers to an aqueous saturated sodium chloride solution.

DMF refers to N,N-dimethylformamide.

THF refers to tetrahydrofuran.

CBZ refers to carbobenzyloxy.

n-BuLi refers to n-butyl lithium

SG refers to silica gel.

IR refers to infrared spectroscopy.

¹H-NMR refers to nuclear (proton) magnetic resonance spectroscopy, chemical shifts are reported in ppm (δ) downfield from tetramethylsilane.

φ refers to phenyl (C₆H₅).

MS refers to mass spectrometry expressed as m/e or mass/charge unit.

[M + H]⁺ refers to the positive ion of a parent plus a hydrogen atom.

EI refers to electron impact.

CI refers to chemical ionization.

FAB refers to fast atom bombardment.

Ether refers to diethyl ether.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

When solvent pairs are used, the ratios of solvents used are volume/volume (v/v).

When the solubility of a solid in a solvent is used the ratio of the solid to the solvent is weight/volume (wt/v).

Examples Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### Preparation 1 N,N-[1,2-Bis(dimethylsilyl)ethane]-3,5-difluoroaniline

A flame dried, 500 mL, 3-necked, round-bottomed flask is fitted with a Claisen tube, mechanical stirrer and a low-temperature thermometer. The flask is loaded with 8.64 g (66.9 mmol) of the starting difluoroaniline. The Claisen tube is fitted with a gas inlet adapter and a rubber septa. The starting material is placed under an atmosphere of nitrogen and 135 mL of anhydrous THF is added. The resulting solution is cooled in a dry ice-isopropanol bath and stirred mechanically. To the cold reaction solution is slowly added 88 mL (141 mmol, 2.1 eq) of n-BuLi (1.6M in hexanes). The reaction temperature is maintained between -70 and -40°C during the addition. After an additional 20 min, 14.4 g (66.9 mmol, leq) of 1,2-bis(chlorodimethylsilyl)ethane in 135 mL of anhydrous THF is slowly added. The homogenous solution is allowed to stir for an additional 45 min and then warmed to room temperature. The reaction is carefully quenched with 200 mL of water and extracted with diethyl ether (4x200 mL). The combined organics are washed with brine, dried (sodium sulfate), filtered and concentrated under reduced pressure to a tan, waxy solid (19.6 g). The crude material is purified by sublimation (5 torr, 40°C). 11.8 g (65%) of the title compound is recovered as a white solid (MP: 71-72°C).

| | | | |
|---|---|---|---|
| Anal. Calc'd for C₁₂H₁₉F₂NSi₂: | C, 53.10; | H, 7.05; | N, 5.15 |
| Found: | C, 52.04; | H, 7.33; | N, 4.99 |

FTIR(neat): cm⁻¹ 2958, 2928, 1626, 1581, 1476, 1453, 1345, 1248, 995.

MS(EI): m/z(rel. int.) 271[M⁺](28), 256(100), 228(11), 73(10).

¹H-NMR(CDCl₃): δ 6.36(dd, 2H, J_{HF}=8.1 Hz, J_{HH}=2.1 Hz), 6.29(dd, 1H, J_{HF}=9.0 Hz, J_{HH}=2.1 Hz), 0.85(s, 4H), 0.25(s, 12H).

### Preparation 2 4-(3-Pyridyl)-3,5-difluoroaniline

A flame dried, 3-necked, round-bottomed flask is loaded with 1.82 g (6.72 mmol) of the protected aniline (Preparation 1) and fitted with a rubber septa, low-temperature thermometer and a gas inlet adapter. The starting material is placed under an atmosphere of nitrogen and 27 mL of anhydrous THF is added. The resulting solution is cooled to -70°C. To the cold solution is added 5.03 mL (8.06 mmol, 1.2 eq) of n-BuLi (1.6M in hexanes) *via* a syringe pump (0.12 mL/min). After the addition is complete, the yellow reaction solution is allowed to stir for an additional 20 min and then 8.06 mL (8.06 mmol, 1.2 eq) of zinc chloride (1.0 M in THF) is added. The reaction is warmed to -40 °C for 15 min. To the yellow solution is added 773 mg (0.672 mmol, 0.10 eq) of Pd(PPh₃)₄ in 50 mL of THF followed by 647 µL (6.72 mmol, 1.0 eq) of 3-bromopyridine. The cooling bath is removed and the reaction is warmed to room temperature. The thermometer is replaced with a condenser and the septa with a glass stopper. The reaction is heated to reflux temperature for 16 h. After this time, the reaction is quenched with 100 mL of 10% HCl(aq). The resulting suspension is vigorously stirred for 0.5 h and then washed with diethyl ether (3x50 mL). The recovered aqueous layer is adjusted to pH 14 with 50% sodium hydroxide (aq) and then extracted with (4x50 mL). The combined organics are washed with brine, dried (sodium sulfate), filtered and concentrated under reduced pressure to yellow solid (935 mg). This crude material is purified by silica gel chromatography (200 g of SG, eluted with 1 L x 5% CH₃CN/CHCl₃, 1 L x 10% CH₃CN/CHCl₃, 1 L x 15% CH₃CN/CHCl₃). 625 mg (43%) of the title compound is recovered as a white solid (MP: 141-142°C).

| | | | |
|---|---|---|---|
| Anal. Calc'd for C₁₁H₈F₂N₂: | C, 64.08; | H, 3.91; | N, 13.59 |
| Found: | C, 63.92; | H, 3.74; | N, 13.24 |

FTIR(mull): cm⁻¹ 3141, 1634, 1460, 1164, 1012, 708.

MS(EI): m/z(rel. int.) 206[M⁺](100), 205(7), 179(5), 158(4), 89(4).

¹H-NMR(CDCl₃): δ 8.67(s, 1H), 8.55(dd, 1H, J=4.9, 1.7 Hz), 7.75(dm, 1H, J=8.1 Hz), 7.35(ddd, 1H, 7.8, 4.9, 0.8 Hz), 6.31(ddd, 2H, J_{HF}=9.8, 1.5 Hz, J_{HH}=3.7 Hz), 4.01(s(b), 2H).

### Preparation 3 N-Carbobenzyloxy-4-(3-pyridyl)-3,5-difluoroaniline

In 60 mL of dry THF is combined 625 mg (3.03 mmol) of the starting amine (Preparation 2) and 382 mg (4.55 mmol, 1.5 eq) of sodium bicarbonate. The resulting mixture is placed under an atmosphere of nitrogen and 394 µL (4.55 mmol, 1.5 eq) of benzylchloroformate is added. The reaction is allowed to stir at room temperature for 16 h. After this time, the reaction is added to 150 mL of methylene chloride and washed with saturated sodium bicarbonate then brine, dried (sodium sulfate), filtered and concentrated under reduced pressure to a white solid (1.09 g). The material is puried by silica gel chromatography (200 g of SG, eluted with 1 L x 3% CH₃CH/CHCl₃, 2 L x 5% CH₃CN/CHCl₃) 990 mg (96%) of the title compound is recovered as a white solid (MP 188-190°C).

FTIR (neat): cm⁻¹ 3030, 1737, 1642, 1478, 1408, 1231, 1029, 723.

| | | | |
|---|---|---|---|
| Anal. calc'd for C₁₉H₁₄F₂N₂O₂: | C, 67.06; | H, 4.15; | N, 8.23 |
| Found: | C, 66.18; | H, 4.22; | N, 7.34 |

MS (EI): m/z (rel. int.): 340 [M⁺] (21), 296 (5), 232 (4), 205 (2), 91 (100), 79 (3).

¹H NMR (CD₃OD): δ 8.59 (s(b), 1H), 8.53-8.51 (m, 1H), 7.95-7.92 (m, 1H), 7.56-7.51 (m, 1H), 7.44-7.31 (m, 5H), 7.28 (d, 2H, J_{HF}=10.4 Hz), 5.21 (s, 2H).

### Preparation 4 N-Allyl-N-carbobenzyloxy-4-(3-pyridyl)-3,5-difluoroaniline

A solution of 543 mg (1.60 mmol) of the starting carbamate (Preparation 3) in 40 mL of anhydrous THF is treated with 128 mg (3.19 mmol. 2 eq) of sodium hydride (60% dispersion in mineral oil). The reaction is kept under an atmosphere of nitrogen at room temperature for 0.5 h. After this time, 691 µL (7.99 mmol, 5 eq) of allyl bromide is added. Stirring is continued at room temperature for 16 h. After this time, the reaction is carefully quenched with 20 mL of water. The layers are separated and the aqueous layer is extracted with ethyl acetate (3 x 25 mL). The combined organics are washed with brine, dried (sodium sulfate), filtered and concentrated under reduced pressure to a dark amber oil. This material is purified by silica gel chromatography (125 g of SG, eluted with 1 L x 3% CH₃CN/CHCl₃) 497 mg (82%) of the title compound is recovered as a yellow solid. An analytical sample is prepared by preparative TLC (SG, 6% CH₃CN/CHCl₃). The analytical sample is recovered as a white solid (MP 92-93°C).

FTIR (mull): cm⁻¹ 3068, 3061, 1710, 1705, 1643, 1635, 1412, 1263, 1024, 733.

| | | | |
|---|---|---|---|
| Anal. calc'd for: C₂₂H₁₈F₂N₂O₂: | C, 69.47; | H, 4.77; | N, 7.36 |
| Found: | C, 68.38; | H, 4.83; | N, 7.19 |

MS (EI): m/z (rel. int.) 380 [M⁺] (15), 336 (4), 309 (3), 245 (4), 91 (100), 65 (6).

¹H NMR (CDCl₃): δ 8.72 (s, 1H), 8.62 (s (b), 1H), 7.99 (d, 1H, J=7.8 Hz), 7.42-7.36 (m, 6H), 7.03 (d, 2H, J_{HF}=9.2 Hz), 5.94 (ddt, 1H, J=11.7, 10.4, 5.2 Hz), 5.23-5.18 (m, 4H), 4.34 (d, 2H, J=5.4 Hz).

### Preparation 5 (±)-5-(Iodomethyl)-3-[4-(3-pyridyl)-3,5-difluorophenyl]-2-oxazolidinone

In 25 mL of chloroform is combined 496 mg (1.31 mmol) of Preparation 4, 1.58 mL (19.6 mmol, 15 eq) of pyridine and 4.97 g (19.6 mmol, 15 eq) of I₂. The resulting mixture is placed under an atmosphere of nitrogen and heated to 50°C with stirring. After 1.5 h, the reaction is decanted into 70 mL of chloroform. The remaining sludge is rinsed with chloroform (3x15 mL). The combined organics are washed with 20% sodium thiosulfate then brine, dried (sodium sulfate), filtered and concentrated under reduced pressure to a yellow, solid foam (491 mg). This material is purified by silica gel chromatography (100 g of SG, eluted with 1L x 0.5% MeOH/CHCl₃, 1 L x 1% MeOH/CHCl₃). 269 mg (49%) of the title compound is recovered as a yellow solid (MP 133-134°C).

FTIR (mull): cm⁻¹ 3130, 1758, 1650, 1414, 1241, 1017, 846.

| | | | |
|---|---|---|---|
| Anal. calc'd for C₁₅H₁₁F₂IN₂O₄: | C, 43.29; | H, 2.66; | N, 6.73 |
| Found: | C, 43.19; | H, 2.56; | N, 6.59 |

¹H NMR (CDCl₃): δ 8.72 (s, 1H), 8.62 (s(b), 1H), 7.79 (d, 1H, J=10.7 Hz), 7.43-7.38 (m, 1H), 7.32 (d, 2H, J_{HF}=9.9 Hz), 4.84-4.72 (m, 1H), 4.19 (dd, 1H, J=8.9, 8.9 Hz), 3.80 (dd, 1H, J=6.0. 9.2 Hz), 3.51 (dd, 1H, J=3.8, 10.5 Hz), 3.39 (dd, 1H, J=8.2, 10.5 Hz).

### Preparation 6 (±)-5-(Azidomethyl)-3-[4-(3-pyridyl)-3,5-difluorophenyl]-2-oxazolidinone

In 15 mL of DMF (dried over 4A sieves) is combined 577 mg (1.31 mmol) of the starting iodide (Preparation 5) and 681 mg (10.5 mmol, 8 eq) of sodium azide. The reaction is placed under an atmosphere of nitrogen and heated to 55°C. After 2 h, the reaction is added to 100 mL of water and then extracted with ethyl acetate (4x25 mL). The combined organics are washed with water then brine, dried (sodium sulfate), filtered and concentrated under reduced pressure to an amber oil (354 mg, 82% crude yield). The crude azide is suitable for reduction without further purification. An analytical sample is prepared by prepatative TLC (SG, 10% CH₃CN/CHCl₃). 22 mg of the title compound is recovered as an off white solid (MP 97-98°C).

FTIR (mull): cm⁻¹ 3483, 2110, 1746, 1640, 1417, 1237, 1064, 716.

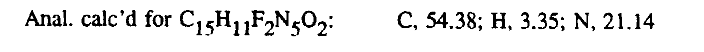

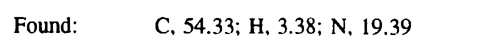

MS (EI): m/z (rel. int.): 331 [M⁺] (100), 274 (14), 258 (23), 232 (15). 217 (22), 190 (22), 43 (14).

¹H NMR (CDCl₃): δ 8.72 (s(b), 1H), 8.63 (s(b), 1H), 7.79 (d, 1H, J=7.9 Hz), 7.42-7.38 (m, 1H), 7.34 (d, 2H, J_{HF}=9.9 Hz), 4.90-4.82 (m, 1H), 4.11 (dd, 1H, J=8.9, 8.9 Hz), 3.88 (dd, 1H, J=6.2, 8.9 Hz), 3.77 (dd, 1H, J=4.4, 13.4 Hz), 3.63 (dd, 1H, J=4.1, 13.3 Hz).

### Preparation 7 (±)-5-(Aminomethyl)-3-[4-(3-pyridyl)-3,5-difluorophenyl]-2-oxazolidinone

In 40 mL of methanol is combined 354 mg (∼1.07 mmol) of the crude azide (Preparation 6) and 50 mg of 10% pd-C. The mixture is purged with nitrogen and then placed under an atmosphere of hydrogen (1 atm). The reaction is allowed to stir at room temperature for 16 h. After this time, the reaction is filtered and concentrated under reduced pressure to an amber solid foam (291 mg, ∼89% crude). The crude amine is suitable for acetylation without further purification. An analytical sample is prepared by preparative TLC (SG, 10% MeOH/CHCl₃). The title compound is recovered as a white solid (MP 143-145°C).

FTIR (neat) cm⁻¹ 3368 (b), 1757, 1646, 1412, 1244, 1025, 714.

| | | | |
|---|---|---|---|
| Anal. calc'd for C₁₅H₁₃F₂N₃O₂: | C, 59.02; | H, 4.29; | N, 13.76 |
| Found: | C, 57.94; | H, 4.21; | N, 13.13 |

MS(EI): m/z (rel. int.) 305 [M⁺] (67), 276 (15), 233 (66), 219 (12), 44 (13), 29 (100).

¹H NMR (CDCl₃): δ 8.71 (s, 1H), 8.62 (dd, 1H, J=1.6, 4.9 Hz), 7.79 (d, 1H, J=8.1 Hz), 7.39 (dd, 1H, J=4.9, 8.0 Hz), 7.33 (d, 2H, J_{HF}=10.0 Hz), 4.794.70 (m, 1H), 4.06 (dd, 1H, J=8.7, 8.7 Hz), 3.92 (dd, 1H, J=6.7, 8.6 Hz), 3.18 (dd, 1H, J=3.9, 14 Hz), 2.98 (dd, 1H J=5.3, 14 Hz), 1.52 (s (b), 2H).

### Preparation 8 4(4-pyridyl)-3,5-difluoroaniline

In a manner similar to that described previously for Preparation 2, 2.00 g (7.38 mmol) of Preparation 1 is transmetallated and combined with Pd(PPh₃)₄ (0.1 eq) and 4-bromopyridine (1 eq). The 4-bromopyridine is freshly prepared from the HCI salt as described below. The salt is neutralized in excess saturated sodium bicarbonate. The free base is then extracted with diethyl ether. The combined organics are dried over magnesium sulfate for 15 min, filtered, then concentrated under reduced pressure to a yellow oil. The free base is stored in a stoppered flask, under nitrogen and frozen in dry ice prior to use. The free base quickly decomposes at room temperature. The reaction is worked up as previously described. 889 mg of the crude product is isolated. ¹H NMR showed this material to be a mixture of desired product and its zinc chloride complex (∼1:1). An analytical sample is prepared by preparative TLC (SG, 10% CH₃CN/CHCl₃).

¹H NMR (CDCl₃): δ 8.63 (dd, 2H, J=1.6, 4.6 Hz), 7.38 (dd, 2H, 1.7, 4.7 Hz), 6.30 (ddd, 2H, J_{HF}=6.6, 10.3 Hz, J_{HH}=8.7 Hz), 4.06 (s(b), 2H).

### Preparation 9 N-Carbobenzyloxy-4-(4-pyridyl)-3,5-difluoroaniline

In a manner to that previously described for Preparation 3, 236 mg (1.15 mmol) of the starting amine (Preparation 8) is converted to the carbamate derivative. The crude product is purified by silica gel chromatography (100 g of SG, eluted with a 3-5% CH₃CN/CHCl₃ gradient). 171 mg (44%) of the title compound is recovered as a white solid.

MP: 185-186°C.

FTIR (neat): cm⁻¹ 1743, 1642, 1605, 1254, 1072.

HRMS: calc'd for C₁₉H₁₄F₂N₂O₂: 340.1023. Found: 340.1029.

MS (EI): m/z (rel. int.): 340 [M⁺] (12), 232 (16), 108 (7), 91 (100), 79 (9), 43 (28).

¹H NMR (CDCl₃): δ 8.67 (s(b), 2H), 7.43-7.38 (m, 7H), 7.15 (d, 2H, J_{HF}=9.9 Hz), 5.23 (s, 2H).

### Preparation 10 N-Allyl-N-carbobenzyloxy-4-(4-pyridyl)-3,5-difluoroaniline

In a manner similar to that previously described for Preparation 4, 468 mg (1.38 mmol) of the starting carbamate (Preparation 9) is allylated. The crude product is purified by silica gel chromatography (125 g of SG, eluted with 2 L x 3% CH₃CN/CHCl₃). 216 mg (41%) of the title compound is recovered as yellow solid.

MP: 82-83°C.

FTIR (neat): cm⁻¹ 1713, 1635, 1598, 1396, 1312, 1235, 1028.

Anal. calc'd for C₂₂H₁₈F₂N₂O₂: C, 69.47; H, 4.77; N, 7.36. Found: C, 69.06; H, 4.80; N, 7.31.

MS (EI): m/z (rel. int.) 380 [M⁺] (16), 330 (6), 246 (26), 219 (13), 91 (100), 40 (16).

¹H NMR (CDCl₃): δ 8.69 (d, 2H, J=5.3 Hz), 7.41-7.33 (m, 7H), 7.03 (d, 2H, J_{HF}=9.6 Hz), 5.92 (ddt, 1H, J=11.8, 10.4, 5.3 Hz), 5.25-5.17 (m, 4H), 4.34 (d, 2H, J=5.4 Hz).

### Preparation 11 (±)-5-Iodomethyl-3-[4-(4-pyridyl)-3,5-difluorophenyl]-2-oxazolidinone

In a manner to that previously described, 241 mg (0.634 mmol) of the cyclization precursor (Preparation 10) is converted to the oxazolidinone iodide. The crude product is not purified further.

¹H NMR (CDCl₃): δ 8.70 (s(b), 2H), 7.44 (d, 2H, J=5.5 Hz), 7.32 (d, 2H, J_{HF}=10.2 Hz), 4.85-4.73 (m, 1H), 4.19 (dd, 1H, J=9.0, 9.0 Hz), 3.80 (dd, 1H, J=6.1, 9.2 Hz), 3.50 (dd, 1H, J=6.1, 10.5 Hz), 3.40 (dd, 1H, J=8.0, 10.5 Hz).

### EXAMPLE 1 (±)-5-(Acetamidomethyl)3-[4-(3-pyridyl)-3,5-difluorophenyl]-2-oxazolidinone

In 10 mL of anhydrous methylene chloride is combined 135 mg (0.433 mmol) of the starting amine (Preparation 7), 143 µL (1.77 mmol, 4 eq.) of pyridine and 167 µL (1.77 mmol, 4 eq.) of acetic anhydride. The reaction is allowed to stir at room temperature under nitrogen. After 2.5 h the reaction is added to 30 mL of methylene chloride and washed with saturated sodium bicarbonate then brine, dried (sodium sulfate), filtered and concentrated under reduced pressure to a yellow solid (138 mg). This material is purified by silica gel chromatography (70 g of SG, eluted with 1-4% MeOH/CHCl₃ gradient), 109 mg (71%) of the title compound is recovered as a white solid (MP 218-219°C).

FTIR (mull): cm⁻¹ 3347, 1742, 1679, 1648, 1563, 1409, 1247, 1022, 755.

| | | | |
|---|---|---|---|
| Anal. calc'd for C₁₇H₁₅F₂N₃O₃: | C, 58.79; | H, 4.35; | N, 12.10 |
| Found: | C. 57.29; | H, 4.35; | N, 11.73 |

MS (EI): m/z (rel. int.) 347 [M⁺] (100), 303 (47), 275 (42), 244 (37), 219 (61), 73 (36), 56 (52).

¹H NMR (CDCl₃): δ 8.68 (s(b), 1H), 8.59 (s(b), 1H), 7.85 (d, 1H, J=7.9 Hz), 7.48-7.41 (m, 2H), 7.31 (ddd, 2H, J_{HF}=3 Hz, 13.2 Hz, J_{HH}=7.7 Hz), 4.87-4.79 (m, 1H), 4.10 (dd, 1H, J=9.1, 9.1 Hz), 3.82 (dd, 1H, J=6.7, 9.2 Hz), 3.72-3.57 (m, 2H), 2.03 (s, 3H).

### EXAMPLE 1a (±)-5-(Acetamidomethy)-3-[4-(3-pyridyl)-3,5-difluorophenyl]-2-oxazolidinone, methanesulfonic acid salt

In 4 mL of methanol is combined 74 mg (0.21 mmol) of (±)-5-(acetamidomethyl)3-[4-(3-pyridyl)]-3,5-difluorophenyl-2-oxazolidinone (EXAMPLE 1) and 14 µL (0.21 mmol) of CH₃SO₃H. The mixture is warmed to reflux temperature for 3 min and the reaction becomes homogeneous. The reaction is cooled to room temperature and concentrated under reduced pressure then *in vacuo* to an amber, gummy solid. The recovered solid is triturated with and the recovered yellow solid (80 mg) is dissolved in 8 mL of water, filtered and lyophilized. 55 mg (58%) of the title compound is recovered as a yellow, hygroscopic solid (MP 203-205°C).

### EXAMPLE 2 (±)-5-(acetamidomethyl)3-[4-(4-pyridyl)]-3,5-difluorophenyl-2-oxazolidinone

In 10 mL of DMF (dried over 4A sieves) is combined 213 mg of Preparation 11 and 166 mg (2.56 mmol, ∼5 eq) of sodium azide. The reaction is placed under an atmosphere of nitrogen and heated to 55°C. After 2 h, the reaction is complete by TLC (6% CH₃CN/CHCL₃), The reaction is added to 50 mL of water and extracted with ethyl acetate (5 x 25 mL). The combined organics are washed with water, then brine, dried (sodium sulfate), filtered and concentrated under reduced pressure to an amber oil (CAUTION: Azides are known to decompose explosively). The crude material is combined with 20 mL of methanol and 30 mg of 10% Pd-C. The reaction is purged with nitrogen then placed under an atmosphere of hydrogen. After 16 h, the reaction is filtered and concentrated under reduced pressure to a white solid. This material is combined with 64 µL of acetic anhydride and 55 µL of pyridine in 11 mL of methylene chloride. The reaction is allowed to stir at room temperature under nitrogen for 12 h. After this time, the reaction is added to 50 mL of methylene chloride and washed with saturated sodium bicarbonate then brine, dried (sodium sulfate), filtered and concentrated under reduced pressure to an amber oil. The final product is purified by silica gel chromatography (100 g of SG, eluted with a 1-4% MeOH/CHCl₃ gradient). 103 mg of the title compound is recovered as a yellow solid (47% over 4 steps) (MP 147-149°C.).

FTIR (neat): cm⁻¹ 1741, 1651, 1646, 1412, 1246, 1027, 745.

| | | | |
|---|---|---|---|
| Anal. calc'd for C₁₇H₁₅F₂N₃O₃: | C, 58.79; | H, 4.35; | N, 12.10 |
| Found: | C, 57.53; | H, 4.48; | N, 11.65 |

MS (EI): m/z (rel. int.) 347 [M⁺] (28), 303 (37), 243 (46), 219 (44), 206 (57), 58 (71), 29 (100).

¹H NMR (CDCl₃): δ 8.70 (d, 2H, J=5.6 Hz), 7.42 (d, 2H, J=6.1 Hz), 7.29 (d, 2H, J_{HF}=10.3 Hz), 6,10 (s(b), 1H), 4.90-4.78 (m, 1H), 4.08 (dd, 1H, J=9.1, 9.1 Hz), 3.82 (dd, 1H, J=6.7, 9.2 Hz), 3.73-3.67 (m, 2H), 2.05 (s, 3H).

### EXAMPLES 3-54

Following the general procedure of Preparations 1-11 and the above EXAMPLES, and (i) starting with the aniline REAGENT (I) listed below for each example, and (ii) using the appropriate aryl or heteroaryl iodide, bromide, triflate or fluorosulfonate for the palladium-mediated coupling reaction (IV → V), the TITLE COMPOUND for the respective EXAMPLE is obtained.

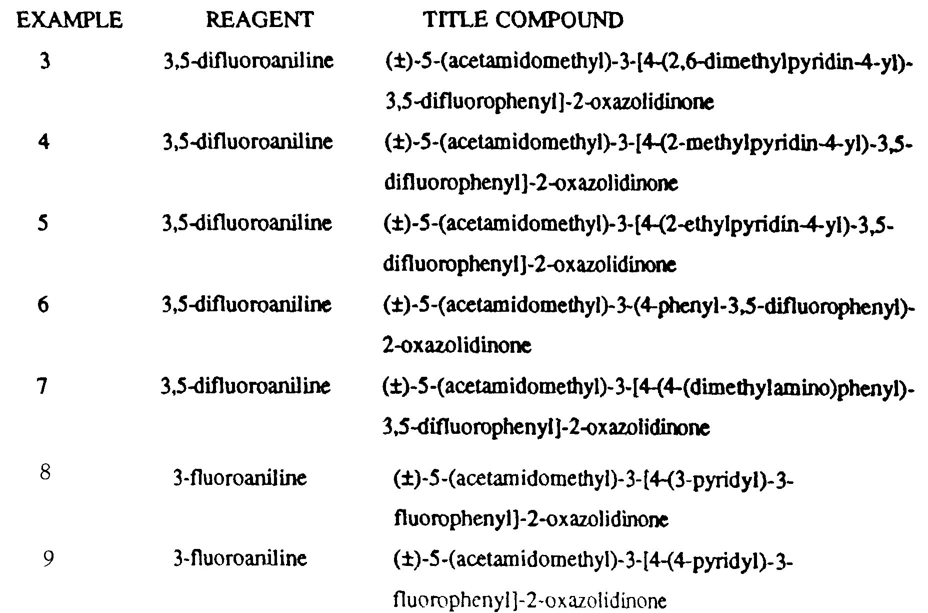

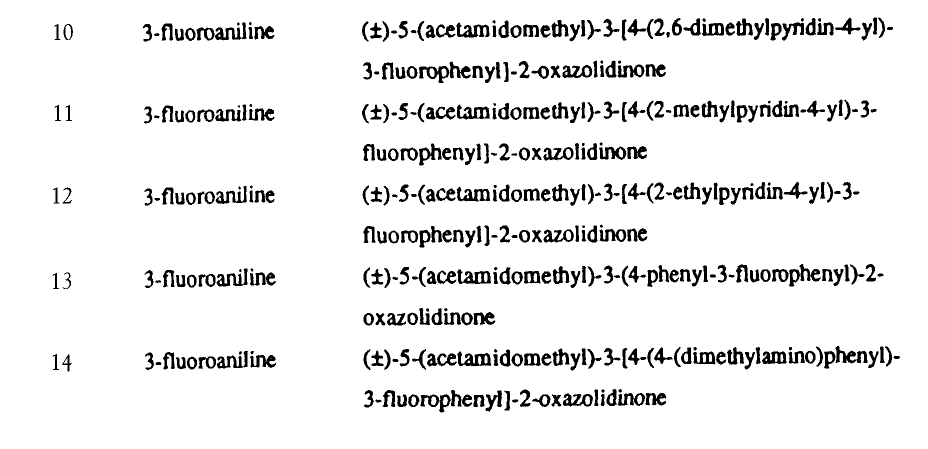

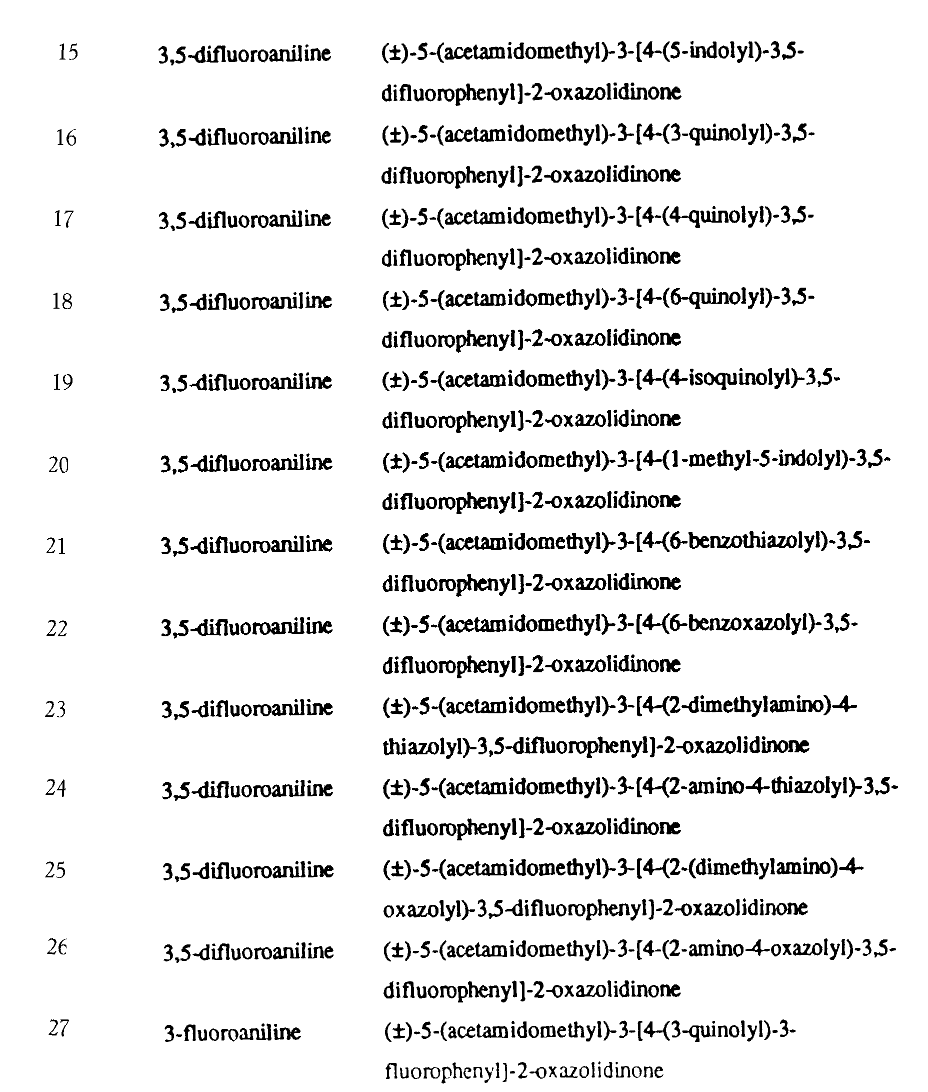

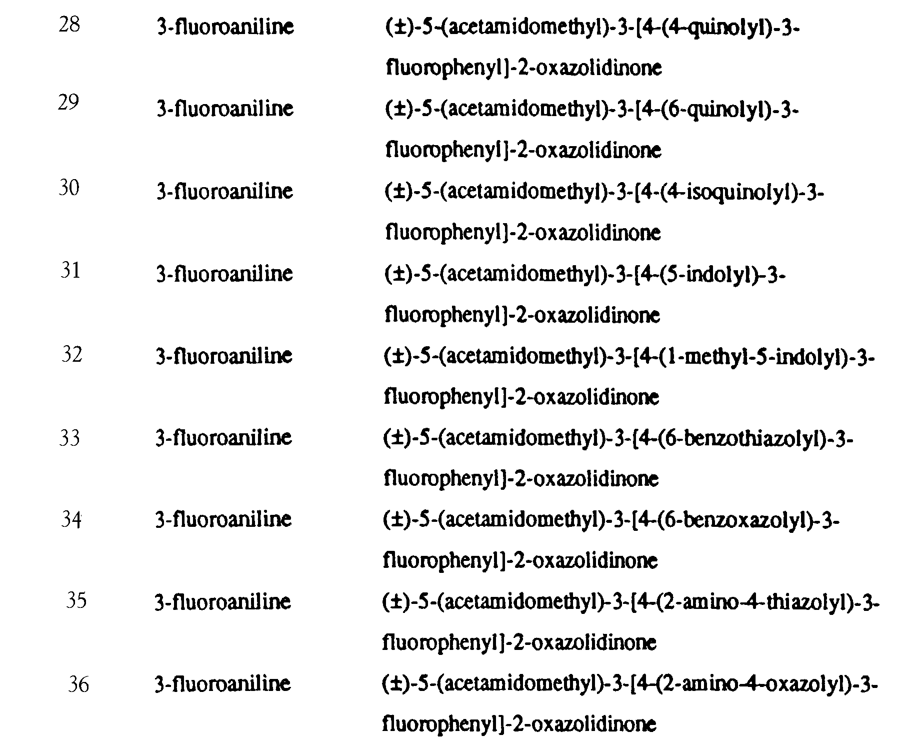

### Preparation 12 (R)-[3-(3-fluorophenyl)-2-oxo-5-oxazolidinyl]methyl butyrate

A mixture of lithium bromide (0.181 g, 2.08 mmol), tri-*n*-butylphosphine oxide (0.454 g, 2.08 mmol), and dry *o*-xylene (10 mL) is azeotropically dried for 1 h. After cooling below the reflux point, a solution of (*R*)-glycidyl butyrate (5.000 g, 34.68 mmol) and 3-fluorophenyl isocyanate (4.755 g or 3.96 mL, 34.68 mmol) in dry *o*-xylene (10 mL) is added over 10 min to the hot solution (some refluxing observed during the addition). When the addition is complete, the solution is heated to reflux for 2 h and then allowed to cool to room temperature. The solvent is removed in vacuo and the residue chromatographed over silica gel, eluting with hexane/ethyl acetate (6:1, 4:1, and then 2:1), to afford 8.758 g (90%) of the title compound as a colorless syrup with the following characteristics:

[α]²⁵ _{D} -46.7° (*c* 1.0, CHCl₃).

IR (mineral oil mull): 1758, 1615, 1591, 1498, 1229, 1197, 1169 cm⁻¹.

¹H-NMR (CDCl₃, 300 MHz) δ 7.44 ("dt", J = 11.2, 2.3 Hz, 1H), 7.34 ("dt", J = 8.3, 6.5 Hz, 1H), 7.23 (ddd, J = 8.3, 2.1, 0.9 Hz, 1H), 6.86 (dddd, J = 8.2, 8.2, 2.5, 0.9 Hz, 1H), 4.88 (m, 1H), 4.39 (dd, J = 12.3, 3.8 Hz, 1H), 4.32 (dd, J = 12.3, 4.7 Hz, 1H), 4.13 ("t", J = 9.0 Hz, 1H), 3.82 (dd, J = 9.0, 6.1 Hz, 1H), 2.33 (t, J = 7.3 Hz, 2H), 1.63 (m, 2H), 0.92 (t, J = 7.4 Hz, 3H).

MS *m/z* (relative intensity): 281 (33.1, M⁺), 193 (9.9), 180 (3.3), 150 (28.7). 148 (68.6), 137 (59.3), 123 (41.7), 95 (38.3), 43 (100).

HRMS *m/z*: 281.1068 (calc'd for C₁₄H₁₆FNO₄: 281.1063).

Anal. calc'd for C₁₄H₁₆FNO₄: C, 59.78; H, 5.73; N, 4.98.

Found: C, 59.98; H, 5.72; N, 4.88.

### Preparation 13 (R)-3-(3-fluorophenyl)-5-(hydroxymethyl)-2-oxooxazolidine

A solution of Preparation 12 (2.789 g, 9.91 mmol) in methanol (10 mL) is treated with a 25 wt. % solution of sodium methoxide in methanol (57 µL, 0.99 mmol) at ambient temperature. After 45 min TLC (5% methanol/chloroform) reveales the starting material is consumed. The reaction mixture is carefully quenched by the addition of 1 N HCl (0.99 mL, 0.99 mmol) and then concentrated in vacuo. Chromatography of the crude product over silica gel, eluting first with 1:1 hexane/ ethyl acetate and then ethyl acetate, affords 1.903 g (91%) of the title compound as a white solid with the following characteristics:

MP: 106.5-107.5°C.

[α]²⁵ _{D} -66.8° (*c* 1.1, CH₃CN).

IR (mineral oil mull): 3520, 1724, 1612, 1590, 1496, 1428, 1420, 1232, 1199 cm⁻¹.

¹H-NMR (CDCl₃, 300 MHz) δ 7.44 ("dt", J = 11.3, 2.3 Hz, 1H), 7.32 ("dt", J = 8.3, 6.5 Hz, 1H), 7.23 (ddd, J = 8.3, 2.1, 1.0 Hz, 1H), 6.84 (dddd, J = 8.2, 8.2, 2.5, 1.0 Hz, 1H), 4.77 (m, 1H), 4.07-3.96 (m, 3H), 3.76 (dd, J = 12.7, 3.9 Hz, 1H), 2.44 (br s, 1H).

MS *m/z* (relative intensity): 211 (100, M⁺), 180 (6.8), 136 (34.3), 124 (84.7), 95 (71.6).

HRMS *m/z* 211.0641 (calc'd for C₁₀H₁₀FNO₃: 211.0645).

Anal. calc'd for C₁₀H₁₀FNO₃: C. 56.87; H, 4.77; N, 6.63.

Found: C, 56.85; H, 4.94; N, 6.56.

The enantiomeric excess of the oxazolidinone alcohol is determined by reacting it with (*R*)-(+)-α-methoxy-α-(trifluoromethyl)phenylacetic acid (DCC, DMAP, CH₂Cl₂, rt), and examining the ¹H-NMR spectrum of the resultant Mosher ester. The %ee is estimated to be ≥ 95%.

### Preparation 14 (R)-3-(3-fluorophenyl)-5-(hydroxymethyl)-2-oxooxazolidine

A solution of N-(carbobenzyloxy)-3-fluoroaniline (1.000 g, 4.08 mmol) in dry tetrahydrofuran (10 mL) is cooled with a dry ice/acetone bath to ca. -78°C and then *n*-butyllithium (1.87 mL of a 1.6 M solution in hexanes, 2.91 mmol) is added. (*R*)-glycidyl butyrate (0.420 g or 0.413 mL, 2.91 mmol) is then added via syringe and the cooling bath allowed to dissipate overnight, with the reaction mixture reaching ambient temperature. The reaction mixture is quenched by the careful addition of saturated aqueous ammonium chloride, the entire mixture transferred to a separatory funnel with dichloromethane washings, and the mixture extracted with dichloromethane. The combined organic extracts are dried over sodium sulfate, filtered and concentrated in vacuo to give an oil which is purified by chromatography over silica gel, eluting with 10% acetonitrile/chloroform containing 1% methanol, to afford 0.555 g (90% based on glycidyl butyrate; 64% based on CBz derivative of the aniline) of the title compound as a white solid identical in all respects to an authentic sample obtained as described in the previous experimental procedure.

### Preparation 15 (R)-[3-(3-fluorophenyl)-2-oxo-5-oxazolidinyl]methyl 4-methylbenzenesulfonate

A solution of Preparation 14 (1.800 g, 8.52 mmol) in dry pyridine (10 mL) is cooled to ca. 5°C and then treated with *p*-toluenesulfonyl chloride (1.706 g, 8.95 mmol). The solution is left at this temperature overnight. TLC (5% methanol/chloroform or 1:1 hexane/ethyl acetate) indicates the starting material is consumed. The reaction mixture is dumped into ice water (30 mL) and the resultant precipitate collected by vacuum filtration through a medium-porosity sintered glass funnel. The collected solids are thoroughly washed with cold water, dried in vacuo, and recrystallized from ethyl acetate/hexane to give 2.743 g (88%) of the title compound as a white solid with the following characteristics:

MP: 114-115°C.

[α]²⁵ _{D} -62.6° (*c* 1.0, CH₃CN).

IR (mineral oil mull): 1751, 1617, 1591, 1499, 1415, 1362, 1227, 1202, 1191, 1172, 1093, 967 cm⁻¹.

¹H-NMR (CDCl₃, 300 MHz) δ 7.78 ("d", J = 8.4 Hz, 2H), 7.38 ("dt", J = 11.2, 2.3 Hz, 1H), 7.36 ("d", J = 7.8 Hz, 2H), 7.33 ("dt", J = 8.3, 6.6 Hz, 1H), 7.16 (ddd, J = 8.3, 2.2, 1.0 Hz, 1H), 6.86 (dddd, J = 8.2, 8.2, 2.5, 1.0 Hz, 1H), 4.84 (m, 1H), 4.29 (dd, J = 11.1, 4.1 Hz, 1H), 4.24 (dd, J = 11.1, 4.6 Hz, 1H), 4.10 ("t", J = 9.1 Hz, 1H), 3.88 (dd, J = 9.2, 6.0 Hz, 1H), 2.46 (s, 3H).

MS *m/z* (relative intensity): 365 (70.6, M⁺), 149 (100), 122 (32.8), 91 (52.8).

HRMS *m/z* 365.0738 (calc'd for C₁₇H₁₆FNO₅S: 365.0733).

Anal. calc'd for C₁₇H₁₆FNO₅S: C, 55.88; H, 4.41; N, 3.83.

Found: C, 55.96; H, 4.38; N, 3.80.

### Preparation 16 (R)-[3-(3-fluorophenyl)-2-oxo-5-oxazolidinyl]methyl azide

A solution of the tosylate (Preparation 15; 2.340 g, 6.40 mmol) in dry DMF (60 mL) is treated with solid sodium azide (3.331 g, 51.23 mmmol) at ambient temperature. The resultant slurry is warmed to 65°C for 4.5 h and then cooled to ambient temperature and left overnight. The reaction mixture is then diluted with ethyl acetate and water, transferred to a separatory funnel, and extracted with ethyl acetate. The combined ethyl acetate extracts are washed thoroughly with water, and then dried (sodium sulfate), filtered, and concentrated in vacuo to give 1.492 g (99%) of the "crude" azide as a white solid which is essentially pure. The following characteristics are noted:

MP: 81-82°C.

[α]²⁵ _{D} -136.5° (*c* 0.9, CHCl₃).

IR (mineral oil mull): 2115, 1736, 1614, 1591, 1586, 1497, 1422, 1233, 1199, 1081, 1049 cm⁻¹.

¹H-NMR (CDCl₃, 300 MHz) δ 7.45 ("dt", J = 11.2, 2.3 Hz, 1H), 7.34 ("dt", J = 8.3, 6.4 Hz, 1H), 7.23 (ddd, J = 8.1, 2.1, 1.0 Hz, 1H), 6.86 (dddd, J = 8.2, 8.2, 2.5, 1.0 Hz, 1H), 4.81 (m, 1H), 4.09 ("t", J = 8.9 Hz, 1H), 3.86 (dd, J = 9.0, 6.2 Hz, 1H), 3.72 (dd, J = 13.2, 4.5 Hz, 1H), 3.60 (dd, J = 13.2, 4.4 Hz, 1H).

MS *m/z* (relative intensity): 236 (59.0, M⁺), 179 (94.9), 136 (59.5), 122 (62.4), 109 (71.8), 95 (100), 75 (40.7).

HRMS *m/z* 236.0708 (calc'd for C₁₀H₉FN₄O₂: 236.0709).

Anal. calc'd for C₁₀H₉FN₄O₂: C, 50.85; H, 3.84; N, 23.72.

Found: C, 50.74; H, 3.76; N, 23.71.

### Preparation 17(S)-N-[(3-(3-fluorophenyl)-2-oxo-5-oxazolidinyl]methyl]acetamide

A solution of the azide (Preparation 16; 8.200 g, 34.71 mmol) in ethyl acetate (100 mL) is treated with 10% palladium on carbon (0.820 g) under nitrogen. The atmosphere is then replaced with hydrogen (balloon) via repeated evacuation and filling. After stirring under hydrogen for 17 h, TLC (5% methanol/chloroform) reveales the azide is consumed. The atmosphere is replaced with nitrogen and then pyridine (6 mL) and acetic anhydride (4.1 mL, 43.40 mmol) are added to the reaction mixture. The reaction mixture is stirred for 1 h at ambient temperature and then filtered through Celite, washing the pad with ethyl acetate. The filtrate is concentrated in vacuo and the residue taken-up in dichloromethane. The addition of diethyl ether affordes a precipitate. After standing in the refrigerator overnight the solids are collected by vacuum filtration, washed with cold hexane, and dried in vacuo to furnish 4.270 g of the title compound as a white solid. Another 3.700 g is obtained from the mother liquors for an overall yield of 91%. In another run, the crude product is purified by chromatography over silica gel, eluting with 5% methanol/chloroform. The following characteristics are noted:

MP: 140.0-140.5°C.

[α]²⁵ _{D} -6.6° (*c* 1.0, CHCl₃).

### Preparation 18 (S)-N-[[3-(3-fluoro-4-iodophenyl)-2-oxo-5-oxazolidinyl]methyl]acetamide

The oxazolidinone intermediate described in Preparation 17 (0.280 g, 1.11 mmol) is dissolved in a mixture of acetic acid (20 mL) and trifluoroacetic acid (5 mL) and then treated with iodine monochloride (2.343 g, 14.43 mmol) at ambient temperature. The dark red-brown mixture is stirred at room temperature under nitrogen. An orange precipitate gradually forms. After ca. 24 h the reaction mixture is diluted with diethyl ether and the solids collected by vacuum filtration through a medium-porosity sintered glass filter, washing with Et₂O. The crude solids are dissolved in hot chloroform (a little methanol is added to aid dissolution), transferred to a separatory funnel, and washed with saturated aqueous sodium bicarbonate, 20% aqueous sodium thiosulfate and brine. The organic phase is dried over sodium sulfate, filtered and concentrated in vacuo to afford 0.295 g (70%) of the title compound as a white solid. The following characteristics are noted:

MP: 185.5-186.5°C.

[α]²⁵ _{D} -37.6° (*c* 1.0, DMF).

### Preparation 19 (±)-N-[[3-[3-fluoro-4-(trimethylstannyl)phenyl]-2-oxo-5-oxazolidinyl]methyl]acetamide

In 1,4-dioxane (10 mL) is combined (±)-N-[[3-(3-fluoro-4-iodophenyl)2-oxo-5-oxazolidinyl]methyl]acetamide (Preparation 18; 0.091 g, 0.24 Mmol), bis(triphenylphosphine)palladium(II) chloride (0.017 g, 0.024 mmol) and hexamethylditin (0.105 g, 0.321 mmol). The reaction mixture is thoroughly purged with nitrogen and heated to reflux temperature for 1.5 h. After this time, the reaction is concentrated under reduced pressure and then purified by silica gel chromatography (10 g of silica gel; eluted with 100 mL each of 0.5, 1, and finally 1.5% methanol/chloroform). After concentration of appropriate fractions, 0.100 g (100%) of the racemic title compound is obtained as a yellow solid with the following characteristics:

MP: 127-130°C.

¹H-NMR (CDCl₃, 300 MHz): δ 7.38-7.33 (m, 2H), 7.19 (dd, J = 8.0, 2.1 Hz, 1H), 6.04 (bs, 1H), 4.83-4.72 (m, 1H), 4.05 (dd, J = 9.1, 9.1 Hz, 1H), 3.76 (dd, J = 9.2, 6.7 Hz, 1H), 3.71-3.59 (m, 2H), 2.02 (s, 3H), 0.34 (d, J = 28.6 Hz, 9H).

MS *m/z* (relative intensity): 415 (3, M⁺), 401 (100), 165 (10), 139 (15), 56 (24), 43 (41).

### EXAMPLE 37 (±)-N-[[3-[3-fluoro-4-(6-quinolyl)phenyl]-2-oxo-5-oxazolidinyl]methyl]acetamide.

(±)-N-[[3-[3-fluoro-4-(trimethylstannyl)phenyl]-2-oxo-5-oxazolidinyl]methyl]acetamide (Preparation 19; 0.367 g, 0.88 mmol), 6-bromoquinoline (0.239 g, 1.15 mmol) and bis(triphenylphosphine)palladium(II) chloride (0.062 g, 0.088 mmol) are combined with DMF (10 mL). The reaction mixture is thoroughly purged with nitrogen and then heated to 80°C under nitrogen After 2 h, little progress is noted by TLC and so the reaction mixture is heated to 95°C for a further 2 h. At this point, TLC revealed the reaction is complete. The mixture is cooled to ambient temperature and concentrated under reduced pressure to give a crude material which is purified by chromatography over silica gel (10 g of silica gel; eluted with methanol/chloroform, 1→4%) to afford 0.136 g (51%) of the racemic title compound as an off-white solid with the following characteristics:

MP: 216-219°C (dec).

IR (internal reflectance): 3411, 3281, 1743, 1657, 1630, 1566, 1521, 1499, 1416, 1226, 1194 cm⁻¹.

¹H-NMR (CDCl₃, 300 MHz): δ 8.95 (dd, J = 4.2, 1.5 Hz, 1H), 8.26-8.20 (m, 2H), 8.00 (br s, 1H), 7.92 (d br t, J = 8.8, 2.0 Hz, 1H), 7.61 (dd, J = 12.9, 2.1 Hz, 1H), 7.57 ("t", J = 8.6 Hz, 1H), 7.47 (dd, J = 8.2, 4.3 Hz, 1H), 7.35 (dd, J = 8.5, 2.1 Hz, 1H), 6.08 (b t, J = 6.0 Hz, 1H), 4.84 (m, 1H), 4.13 ("t", J = 9.0 Hz, 1H), 3.86 (dd J = 9.1, 6.8 Hz, 1H), 3.80-3.62 (m, 2H), 2.05 (s, 3H).

MS *m/z* (relative intensity): 379 (100.0, M⁺), 335 (24.4), 307 (17.0), 276 (42.6), 264 (29.2), 251 (93.8).

### EXAMPLE 38 (S)-N-[[3-[3-fluoro-4-(4-pyridyl)phenyl]-2-oxo-5-oxazolidinyl]methyl]acetamide

A slurry of the oxazolidinone iodide described in Preparation 18 (0.063 g, 0.165 mmol) and bimethyl(4-pyridyl)tin (0.060 g, 0248 mmol) in 1,4-dioxane (5 mL) is degassed by repeated evacuation and filling with nitrogen. Bis(triphenylphosphine)palladium(II) chloride (0.012 g, 0.0165 mmol) is added, the reaction again degassed, and then the mixture is brought to reflux under nitrogen. After 4 h TLC (silica gel, 10% methanol/chloroform) reveales some of the iodide still remaines. The mixture is refluxed a further 20 h, cooled to ambient temperature, and concentrated under reduced pressure. The residue is chromatographed over silica gel, eluting with a little chloroform and then methanol/chloroform (1%, 2%, and then 5%), to afford 0.046 g (84%) of the title compound as a white solid which is identical by ¹H-NMR and chromatographic behavior to a fully characterized racemic sample. The following characteristics are noted for the enantiomerically enriched material:

MP: 190.5-191.0°C.

[α]²⁵ _{D} -16.4° (*c* 0.5, CHCl₃).

The following characteristics are noted for a racemic sample:

MP: 179-180°C.

IR (internal reflectance): 3279, 3063, 1756, 1752, 1657, 1626, 1600, 1542, 1522, 1485, 1412, 1407, 1377, 1222, 1198 cm⁻¹.

¹H-NMR (CDCl₃, 300 MHz) δ 8.67 (dd, J = 6.1, 1.5 Hz, 2H), 7.59 (dd, J = 13.1, 2.2 Hz, 1H), 7.50 ("t", J = 8.5 Hz, 1H), 7.47 (dd, J = 6.1, 1.4 Hz, 2H), 7.33 (dd, J = 8.7, 2.2 Hz, 1H), 6.15 (bt, J = 6.0 Hz, 1H), 4.84 (m, 1H), 4.11 ("t", J = 9.1 Hz, 1H), 3.85 (dd, J = 9.2, 6.7 Hz, 1H), 3.78-3.62 (m, 2H), 2.04 (s, 3H).

MS *m/z* (relative intensity): 329 (39.8, M⁺), 285 (29.3), 257 (14.5), 201 (52.4), 172 (27.1), 73 (27.1), 42 (100.0).

Anal. calc'd for C₁₇H₁₆FN₃O₃: C, 62.00; H, 4.90; N, 12.76.

Found: C, 62.01; H, 4.84; N, 12.82.

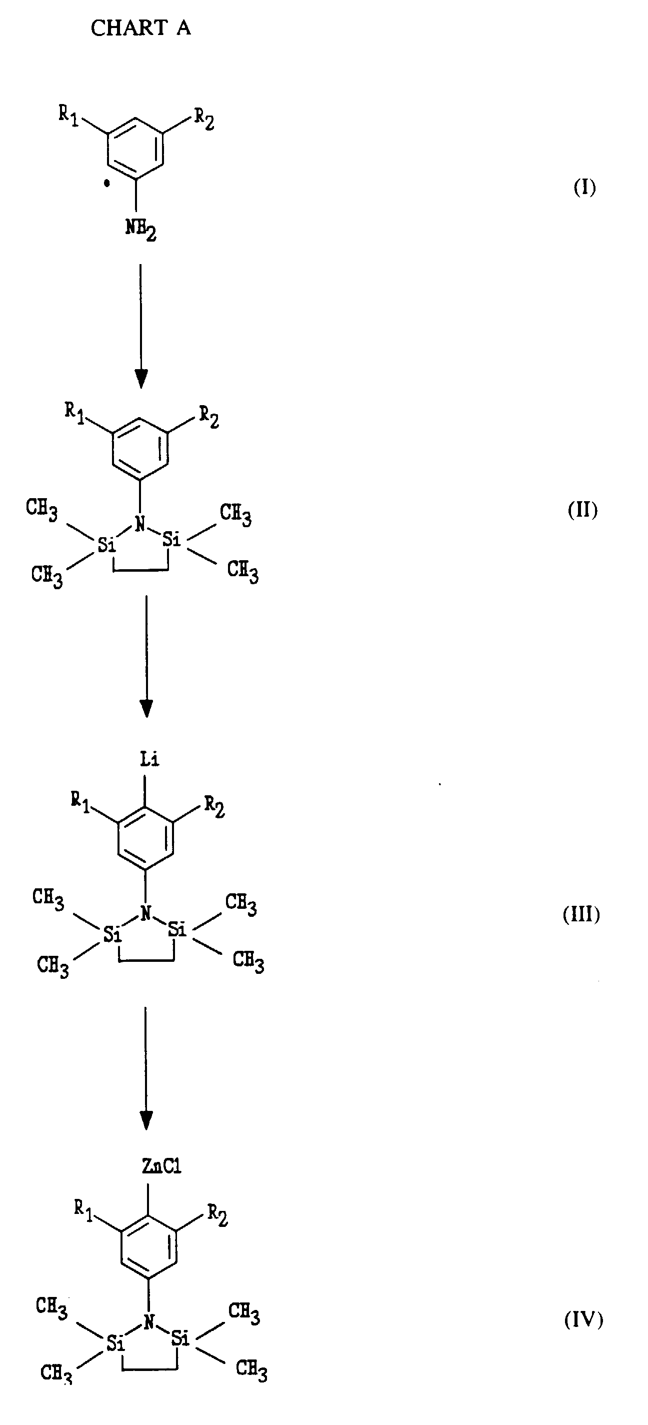

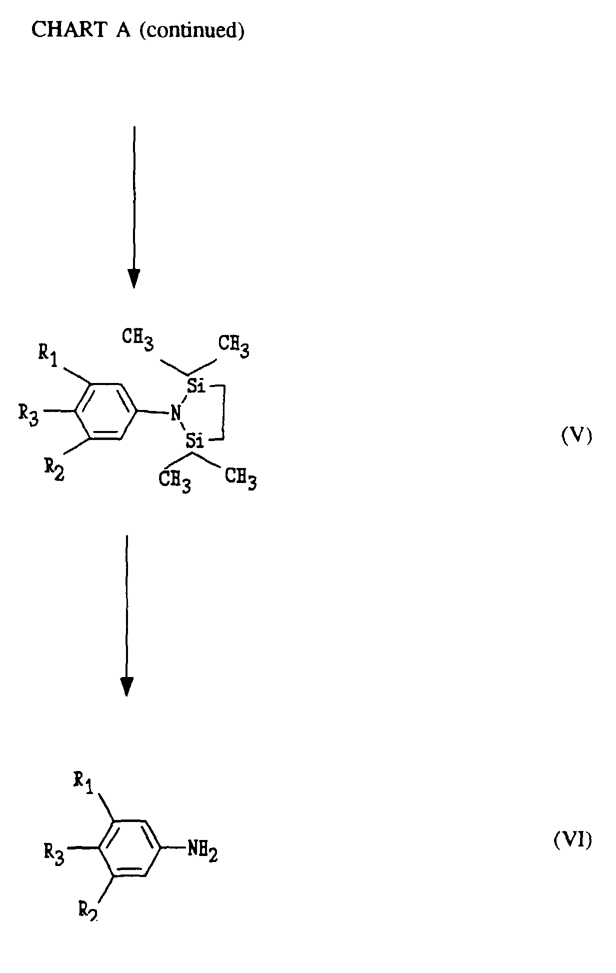

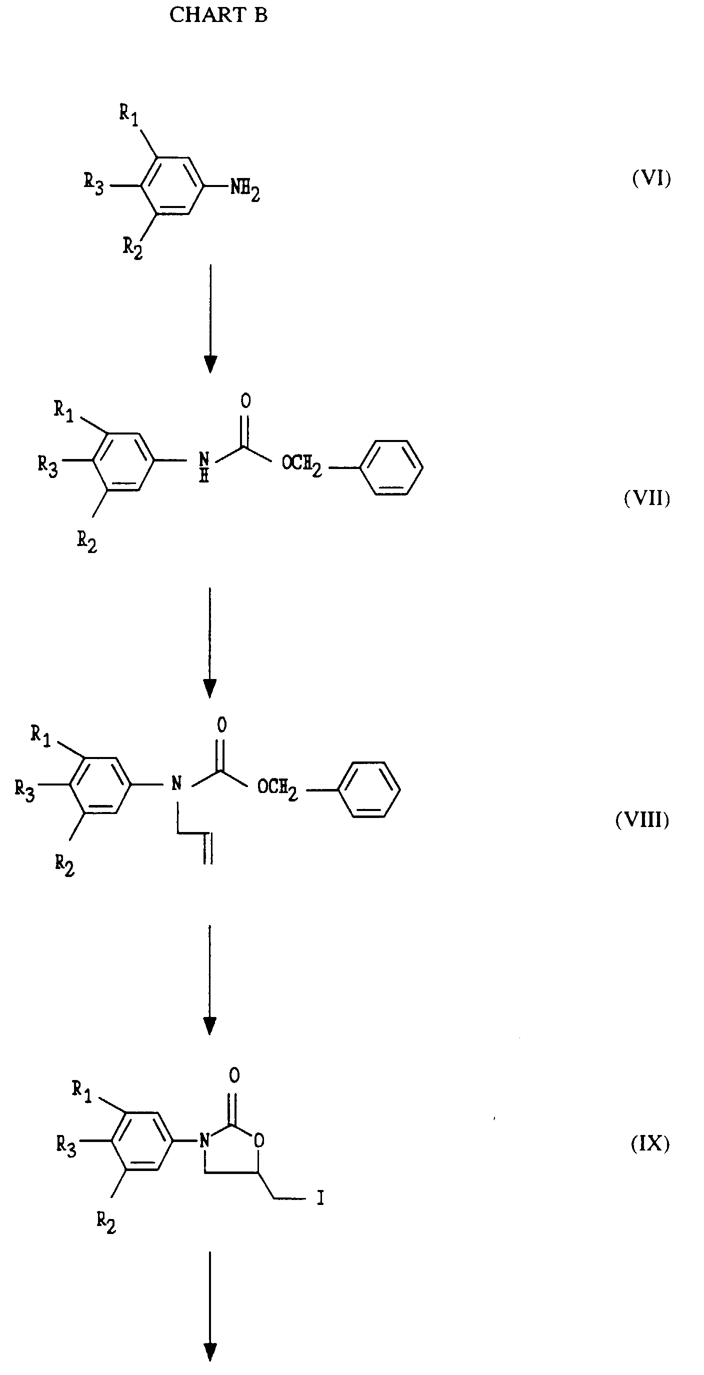

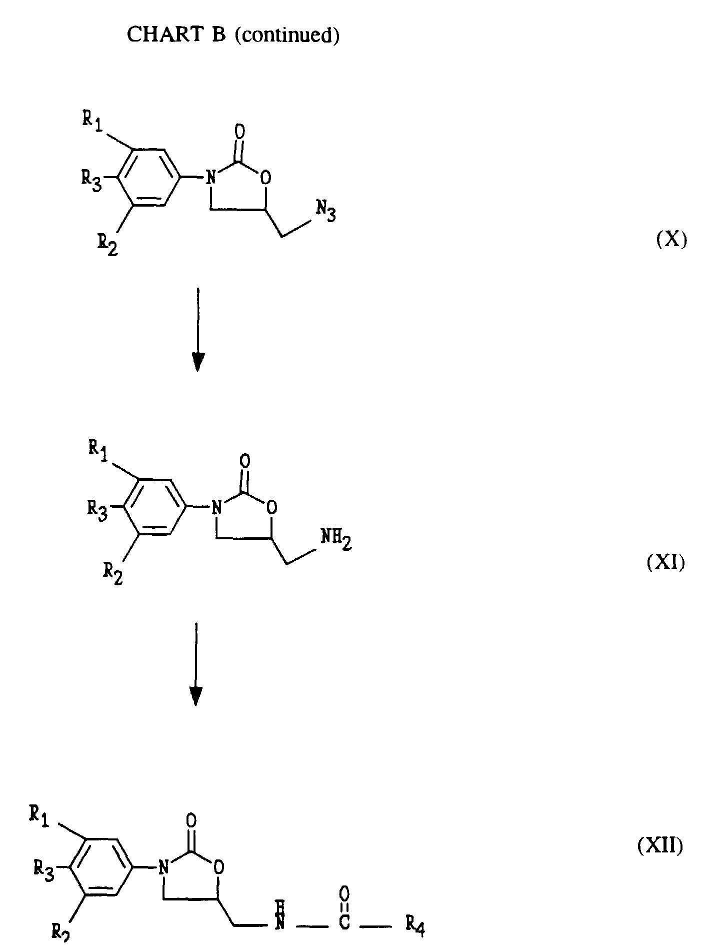

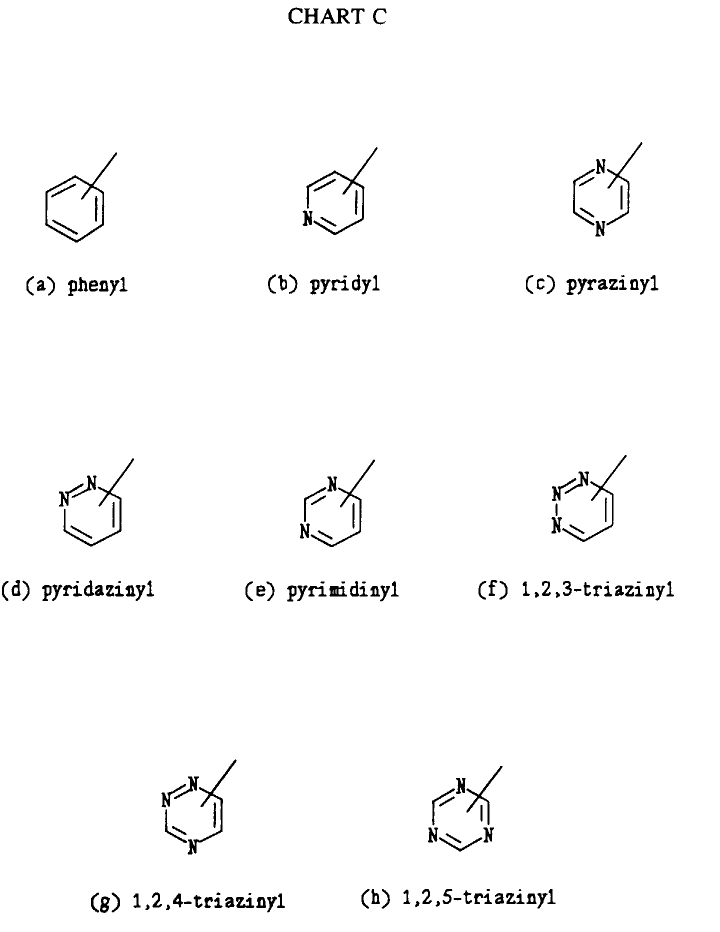

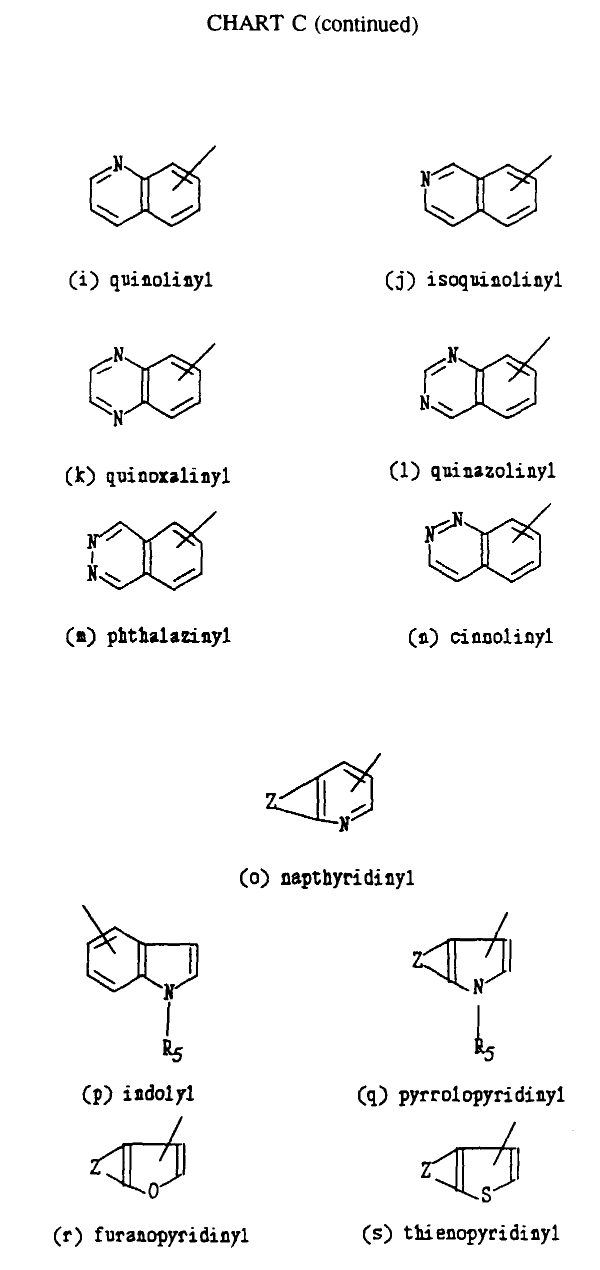

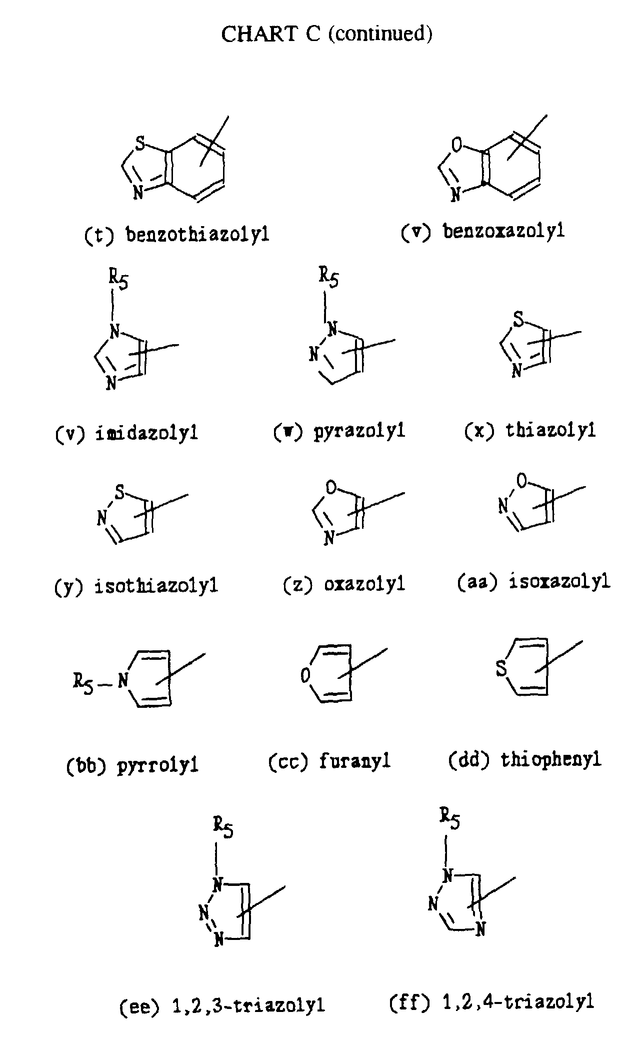

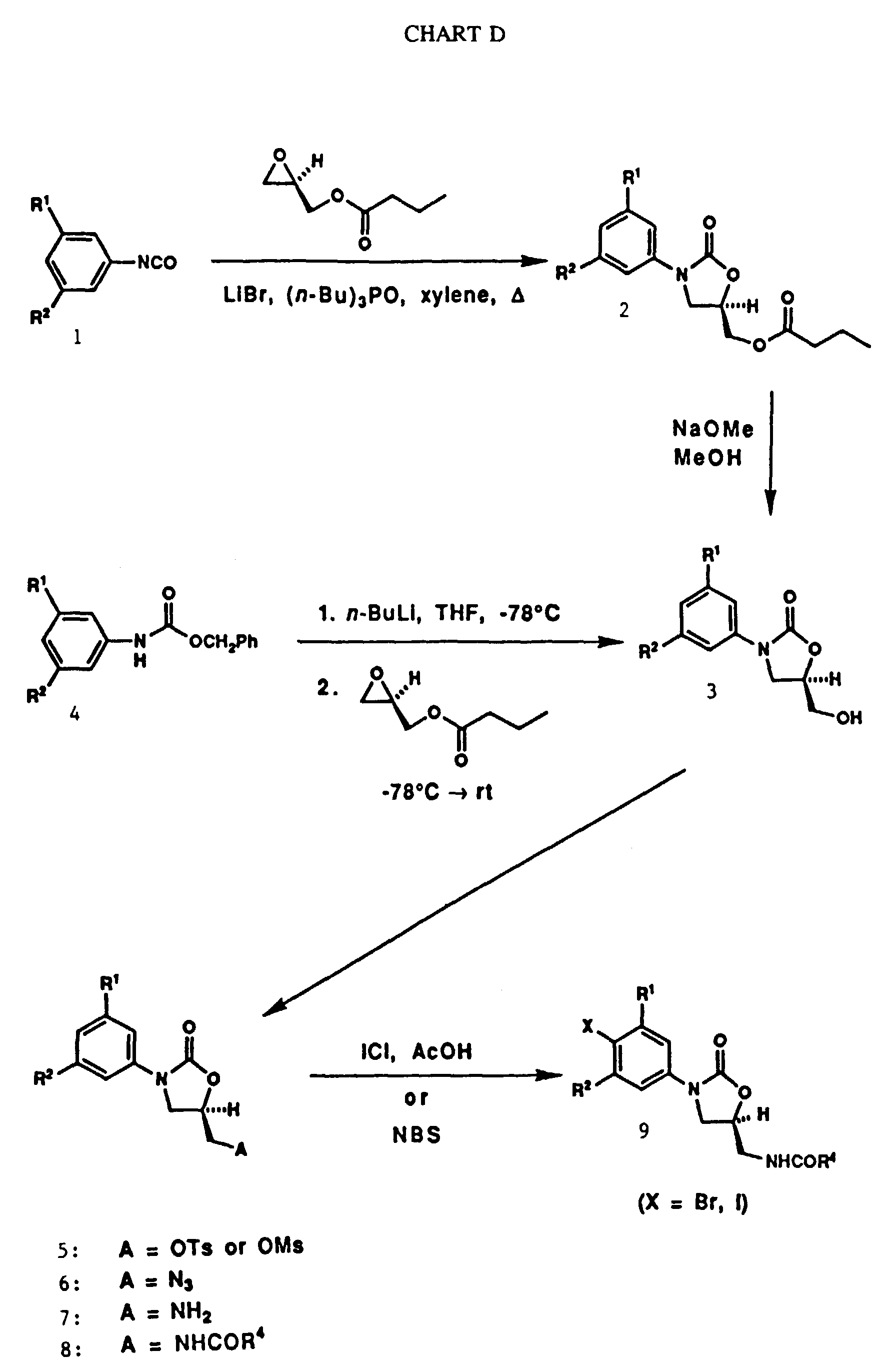

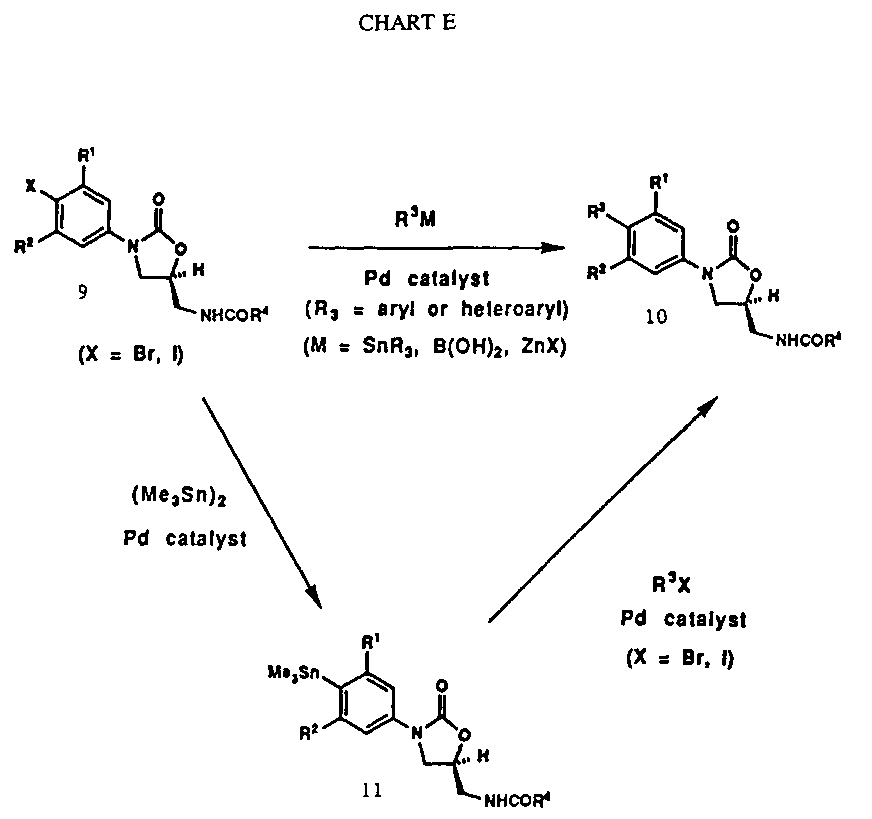

## Claims

1. A substituted aryl- and heteroaryl-phenyl oxazolidinone of Formula (XII) 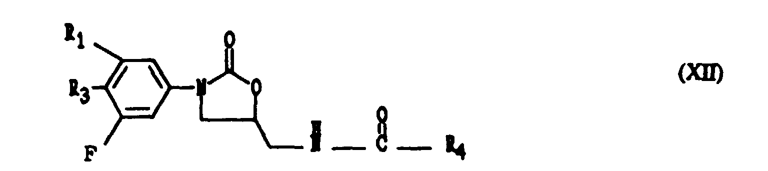 where
(I) R₁ is selected from
(a) -H,
(b) -F,
(c) -Cl,
(d) -CF₃, and
(f) -OCH₃;
(II) R₃ is selected from the group consisting of
(a) phenyl,
(b) pyridyl,
(c) pyrazinyl, (d) pyridazinyl, (e) pyrimidinyl,
(f) 1,2,3-, (g) 1,2,4-, (h) 1,2,5-triazinyl,
(i) quinolinyl, (j) isoquinolinyl,
(k) quinoxalinyl, (l) quinazolinyl, (m) phthalazinyl, (n) cinnolinyl,
(o) naphthyridinyl,
(p) indolyl having nitrogen optionally substituted with R₅₋₁ when R₅₋₁ is
-H,
C₁-C₄ alkyl optionally substituted with one or more halogens,
C₃-C₆ cycloalkyl, or
-C(O)R₅₋₂ where R₅₋₂ is
-H,
C₁-C₄ alkyl optionally substituted with one or more halogens, or phenyl optionally substituted with one or more halogens,
(q) pyrrolopyridinyl having the saturated nitrogen substituted with R₅₋₁ where R₅₋₁ is as defined above, (r) furanopyridinyl, (s) thienopyridinyl,
(t) benzothiazolyl, (u) benzoxazolyl,
(v) imidazolyl having the saturated nitrogen substituted with R₅₋₁ where R₅₋₁ is as defined above,
(w) pyrazolyl having the saturated nitrogen substituted with R₅₋₁ where R₅₋₁ is as defined above.
(x) thiazolyl, (y) isothiazolyl,
(z) oxazolyl, (aa) isoxazolyl,
(bb) pyrroyl having nitrogen substituted with R₅₋₁ where R₅₋₁ is as defined above,
(cc) furanyl, (dd) thiophenyl.
wherein substituents (a)-(dd) are optionally substituted with X and Y,
(ee) 1,2,3-, (ff) 1,2,4-triazolyl having the saturated nitrogen substituted with R₅₋₁ where R₅₋₁ is as defined above,
wherein substituents (ee) and (ff) are optionally substituted with X;
(III) each occurrence of Y is independently selected from
(a) -H,
(b) -Fl, (c) -Cl, (d) -Br, (e) -l,
(f) -R₃₋₁, (g) -OR₃₋₁ where R₃₋₁ is H or C₁-C₄ alkyl, or
(h) -NO₂;
(IV) each occurrence of X is independently selected from
(a) -H,
(h) C₁-C₈ alkyl optionally substituted with
one or more halogens,
-OH,
=O other than at alpha position,
-S(O)ₙR₃₋₂ where R₃₋₂ is C₁-C₄ alkyl or C₃-C₈ cycloalkyl, or
-NR₃₋₃R₃₋₄ where R₃₋₃ and R₃₋₄ are the same or different and are -H, C₁-C₈ alkyl, C₃-C₈ cycloalkyl, -(CH₂)ₜCHOR₃₋₅, -(CH₂)ₜNR₃₋₆R₃₋₇, or taken together are -(CH₂)O(CH₂)-,
-(CH₂)ₜCH(CO)R₃₋₈, or -(CH₂)N(R₃₋₈)(CH₂)₂- where
R₃₋₅ is -H or C₁-C₄ alkyl, or
R₃₋₆ and R₃₋₇ are the same or different and are -H, C₁-C₄ alkyl or taken together are -(CH₂)ᵣ-,
(c) C₂-C₅ alkenyl,
(d) C₃-C₈ cycloalkyl,
(e) -OR₃₋₃ where R₃₋₃ is as defined ahove.
(f) -CN,
(g) -S(O)ₙ-R₃₋₈ where R₃₋₈ is
C₁-C₄ alkyl optionally substituted with
one or more halogens,
-OH,
-CN,
-NR₃₋₃R₃₋₄ where R₃₋₃ and R₃₋₄ are as defined above,
-CO₂R₃₋₅ where R₃₋₅ is as defined above,
C₂-C₄ alkenyl,
-NR₃₋₉R₃₋₁₀ where R₃₋₉ is -H, C₁-C₄ alkyl, or C₃-C₈ cycloalkyl and R₃₋₁₀ is -H, C₁-C₄ alkyl, C₁-C₄ alkenyl, C₃-C₄ cycloalkyl, -OR₃₋₅, or -NR₃₋₆R₃₋₇ where R₃₋₅, R₃₋₆, and R₃₋₇ are as defined above,
-N₃,
-NHC(O)R₃₋₁₁ where R₃₋₁₁ is C₁-C₄ alkyl optionally substituted with one or more halogens,
(h) -S(O)₂-N=S(O)ₚR₃₋₁₄R₃₋₁₅ where R₃₋₁₄ and R₃₋₁₅ are the same or different and are C₁-C₂ alkyl, or taken together are -(CH₂)_{q}-,
(i) -S-C(O)-R₃₋₁₁ where R₃₋₁₁ is as defined above,
(j) tetrazoly,
(k) -NR₃₋₃R₃₋₄ where R₃₋₃ and R₃₋₄ are as defined above.
(l) -N(R₃₋₃)COR₃₋₁₁ where R₃₋₃ and R₃₋₁₁ are as defined above,
(m) -N(R₃₋₃)S(O)ₙR₃₋₁₁ where R₃₋₃ and R₃₋₁₁ are as defined above,
(n) -CONR₃₋₃R₃₋₄ where R₃₋₃ and R₃₋₄ are as defined above,
(o) -C(O)R₃₋₁₆ where R₃₋₁₆ is
-H,
C₁-C₈ alkyl optionally substituted with one or more halogens,
C₁-C₄ alkyl optionally substituted with
-OR₃₋₅,
-OC(O)R₃₋₅,
-NR₃₋₃R₃₋₄,
-S(O)ₙR₃₋₁₇,
C₃-C₈ cycloalkyl, or
C₂-C₅ alkenyl optionally substituted with -CHO or -CO₂R₃₋₅, where R₃₋₃, R₃₋₄, and R₃₋₅ are as defined above and R₃₋₁₇ is C₁-C₄ alkyl or C₃-C₈ cycloalkyl,
(p) -C(=NR₃₋₁₈)R₃₋₆ where R₃₋₁₆ is as defined above and R₃₋₁₈ is -NR₃₋₃R₃₋₄, -OR₃₋₃, or NHC(O)R₃₋₃ where R₃₋₃ and R₃₋₄ are as defined above,
(q) -CR₃₋₁₆(OR₃₋₁₉)OR₃₋₂₀ where R₃₋₁₆ is as defined above and R₃₋₁₉ and R₃₋₂₀ are the same or different and are C₁-C₄ alkyl, or taken together are -(CH₂)ₘ-, 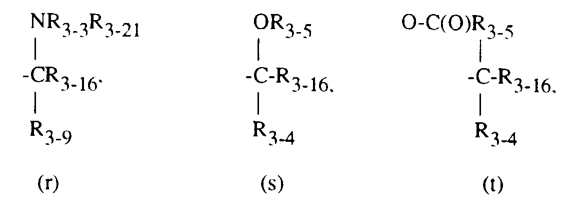 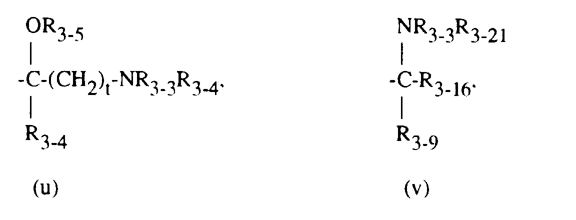 where R₃₋₃, R₃₋₄, R₃₋₅, R₃₋₉, and R₃₋₁₆ are as defined above and R₃₋₂₁ is R₃₋₄ or -NR₃₋₄R₃₋₅ where R₃₋₄ and R₃₋₅ are as defined above,
m is 2 or 3;
n is 0, 1, or 2;
p is 0 or 1;
q is 3, 4 or 5;
t is 1, 2 or 3;
(V) R₄ is selected from the group consisting of
(a) -H,
(b) C₁-C₁₂ alkyl optionally substituted with 1-3 Cl,
(c) C₃-C₁₂ cycloalkyl,
(d) C₅-C₁₂ alkenyl containing one double bond,
(e) phenyl optionally substituted with 1-3 -OH, -OCH₃, -OC₂H₅, -NO₂, -F, -Cl, -Br, -COOH and -SO₃H, -N(R₄₋₁)(R₄₋₂) where R₄₋₁ and R₄₋₂ are the same or different and are -H and C₁-C₅ alkyl,
(f) furanyl,
(g) tetrahydrofuranyl,
(h) 2-thiophene,
(i) pyrrolidinyl,
(j) pyridinyl,
(k) -O-R₄₋₃ where R₄₋₃ is C₁-C₄ alkyl,
(l) -NH₂,
(m) -NHR₄₋₄ where R₄₋₄ is C₁-C₃ alkyl or -φ,
(n) -NR₄₋₄R₄₋₅ where R₄₋₄ is as defined above, and
R₄₋₅ is C₁-C₃ alkyl, or
R₄₋₄ and R₄₋₅ are taken together with the attached nitrogen atom to form a saturated mono-nitrogen C₅-C₇ heterocyclic ring including -O- (morpholine),
(o) -CH₂-OH, or
(p) -CH₂-OR₄₋₆ where R₄₋₆ is C₁-C₄ alkyl or -CO-R₄₋₇ where R₄₋₇ is C₁-C₄ alkyl or -φ;
or a pharmaceutically-acceptable salt thereof.

2. A compound according to claim 1 where R₁ is H or F.

3. A compound according to claim 2 where R₃ is phenyl optionally substituted with X and Y.

4. A compound according to claim 3 which is
(±)-5-(acetamidomethyl)-3-(4-phenyl-3-fluorophenyl)-2-oxazolidinone,
(S)-N-[[3-[3-fluoro-4-(4-pyridyl)phenyl]-2-oxo-5-oxazolidinyl]methyl]acetamide,
(±)-5-(acetamidomethyl)-3-[4-(4-(dimethylamino)phenyl)-3-fluorophenyl]-2-oxazolidinone,
(±)-5-(acetamidomethyl)-3-(4-phenyl-3,5-fluorophenyl)-2-oxazolidinone, or
(±)-5-(acetamidomethyl)-3-[4-(4-(dimethylamino)phenyl)-3,5-difluorophenyl]-2-oxazolidinone.

5. A compound according to claim 2 where R₃ is pyridyl optionally substituted with X and Y.

6. A compound according to claim 5 which is
(±)-5-(acetamidomethyl)-3-[4-(3-pyridyl)-3-fluorophenyl]-2-oxazolidinone,
(±)-5-(acetamidomethyl)-3-[4-(4-pyridyl)-3-fluorophenyl]-2-oxazolidinone,
(±)-5-(acetamidomethyl)-3-[4-(2,6-dimethylpyridin-4-yl)-3-fluorophenyl]-2-oxazolidinone,
(±)-5-(acetamidomethyl)-3-[4-(2-methylpyridin-4-yl)-3-fluorophenyl]-2-oxazolidinone,
(±)-5-(acetamidomethyl)-3-[4-(2-ethylpyridin-4-yl)-3-fluorophenyl]-2-oxazolidinone,
(±)-5-(acetamidomethyl)-3-[4-(3-pyridyl)-3,5-difluorophenyl]-2-oxazolidinone,
(±)-5-(acetamidomethyl)-3-[4-(3-pyridyl)-3,5-difluorophenyl]-2-oxazolidinone, methanesulfonic salt,
(±)-5-(acetamidomethyl)-3-[4-(4-pyridyl)-3,5-difluorophenyl]-2-oxazolidinone,
(±)-5-(acetamidomethyl)-3-[4-(2,6-dimethylpyridin-4-yl)-3,5-difluorophenyl]-2-oxazolidinone,
(±)-5-(acetamidomethyl)-3-[4-(2-methylpyridin-4-yl)-3,5-difluorophenyl]-2-oxazolidinone, or
(±)-5-(acetamidomethyl)-3-[4-(2-ethylpyridin-4-yl)-3,5-difluorophenyl]-2-oxazolidinone.

7. A compound according to claim 2 where R₃ is quinolinyl or isoquinolinyl optionally substituted with X and Y.

8. A compound according to claim 7 which is
(±)-5-(acetamidomethyl)-3-[4-(3-quinolyl)-3-fluorophenyl]-2-oxazolidinone,
(±)-5-(acetamidomethyl)-3-[4-(4-quinolyl)-3-fluorophenyl]-2-oxazolidinone,
(±)-5-(acetamidomethyl)-3-[4-(6-quinolyl)-3-fluorophenyl]-2-oxazolidinone,
(±)-5-(acetamidomethyl)-3-[4-(4-isoquinolyl)-3-fluorophenyl]-2-oxazolidinone,
(±)-5-(acetamidomethyl)-3-[4-(3-quinolyl)-3,5-difluorophenyl]-2-oxazolidinone,
(±)-5-(acetamidomethyl)-3-[4-(4-quinolyl)-3,5-difluorophenyl]-2-oxazolidinone,
(±)-5-(acetamidomethyl)-3-[4-(6-quinolyl)-3,5-difluorophenyl]-2-oxazolidinone, or
(±)-5-(acetamidomethyl)-3-[4-(4-isoquinolyl)-3,5-difluorophenyl]-2-oxazolidinone.

9. A compound according to claim 2 where R₃ is indolyl having nitrogen optionally substituted with R₅ and optionally substituted with X and Y.

10. A compound according to claim 9 which is
(±)-5-(acetamidomethy1)-3-[4-(5-indolyl)-3-fluorophenyl]-2-oxazolidinone,
(±)-5-(acetamidomethyl)-3-[4-(1-methyl-5-indolyl)-3-fluorophenyl]-2-oxazolidinone,
(±)-5-(acetamidomethyl)-3-[4-(5-indolyl)-3,5-difluorophenyl]-2-oxazolidinone, or
(±)-5-(acetamidomethyl)-3-[4-(1-methyl-5-indolyl)-3,5-difluorophenyl]-2-oxazolidinone.

11. A compound according to claim 2 where R₃ is benzothiazolyl or benzoxazolyl optionally substituted with X and Y.

12. A compound according to claim 11 which is
(±)-5-(acetamidomethyl)-3-[4-(6-benzothiazolyl)-3-fluorophenyl]-2-oxazolidinone,
(±)-5-(acetamidomethyl)-3-[4-(6-benzoxazolyl)-3-fluorophenyl]-2-oxazolidinone,
(±)-5-(acetamidomethyl)-3-[4-(6-benzothiazolyl)-3,5-difluorophenyl]-2-oxazolidinone, or
(±)-5-(acetamidomethyl)-3-[4-(6-benzoxazolyl)-3,5-difluorophenyl]-2-oxazolidinone.

13. A compound according to claim 2 where R₃ is thiazolyl or oxazolyl optionally substituted with X and Y.

14. A compound according to claim 13 which is
(±)-5-(acetamidomethyl)-3-[4-(2-amino-4-thiazolyl)-3-fluorophenyl]-2-oxazolidinone,
(±)-5-(acetamidomethyl)-3-[4-(2-amino-4-oxazolyl)-3-fluorophenyl]-2-oxazolidinone,
(±)-5-(acetamidomethyl)-3-[4-(2-dimethylamino)-4-thiazolyl)-3,5-difluorophenyl]-2-oxazolidinone,
(±)-5-(acetamidomethyl)-3-[4-(2-amino-4-thiazolyl)-3,5-difluorophenyl]-2-oxazolidinone,
(±)-5-(acetamidomethyl)-3-[4-(2-dimethylamino)-4-oxazolyl)-3,5-difluorophenyl]-2-oxazolidinone, or
(±)-5-(acetamidomethyl)-3-(4-(2-amino-4-oxazolyl)-3,5-difluorophenyl]-2-oxazolidinone.

15. A compound according to any preceding claim where R₁ is F.

16. A process for making a compound of formula (XII), as defined in claim 1, comprising:
(a) converting a substituted aniline to a stabase derivative;
(b) treating the stabase derivative to form an aryl- or heteroaryl-substituted aniline; and
(c) converting the aryl- or heteroaryl-substituted aniline to a aryl- or heteroaryl-substituted phenyloxazolidinone.

17. A process for making an oxazolidinone iodide, comprising reacting a carbobenzyloxy allyl compound in the presence of an excess of pyridine and iodine, the excess being of equal amounts of 2-20 molar equivalents.

## Patentansprüche

1. Substituiertes Aryl- und Heteroarylphenyloxazolidinon der Formel (XII) 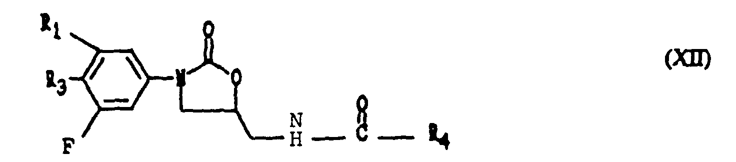 worin
(I) R₁ ausgewählt ist aus
(a) -H,
(b) -F,
(c) -Cl,
(d) -CF₃ und
(f) -OCH₃;
(II) R₃ ausgewählt ist aus
(a) Phenyl,
(b) Pyridyl,
(c) Pyrazinyl, (d) Pyridazinyl, (e) Pyrimidinyl,
(f) 1,2,3-, (g) 1,2,4-, (h) 1,2,5-Triazinyl,
(i) Chinolinyl, (j) Isochinolinyl,
(k) Chinoxalinyl, (l) Chinazolinyl,
(m) Phthalazinyl, (n) Cinnolinyl,
(o) Naphthyridinyl,
(p) Indolyl, wobei der Stickstoff gegebenenfalls durch R₅₋₁ substituiert ist und R₅₋₁ für -H, gegebenenfalls
ein- oder mehrfach halogensubstituiertes
C₁-C₄ Alkyl,
C₃-C₆ Cycloalkyl oder
-C(O)R₅₋₂ mit R₅₋₂ gleich -H,
gegebenenfalls ein- oder mehrfach halogensubstituiertem C₁-C₄ Alkyl oder gegebenenfalls ein- oder mehrfach halogensubstituiertem Phenyl, steht,
(q) Pyrrolopyridinyl, wobei der gesättigte Stickstoff mit R₅₋₁ in der zuvor angegebenen Bedeutung substituiert ist,
(r) Furanopyridinyl, (s) Thienopyridinyl,
(t) Benzothiazolyl, (u) Benzoxazolyl,
(v) Imidazolyl, wobei der gesättigte Stickstoff mit R₅₋₁ in der zuvor angegebenen Bedeutung substituiert ist,
(w) Pyrazolyl, wobei der gesättigte Stickstoff mit R₅₋₁ in der zuvor angegebenen Bedeutung substituiert ist,
(x) Thiazolyl, (y) Isothiazolyl,
(z) Oxazolyl, (aa) Isoxazolyl,
(bb) Pyrroyl, wobei der Stickstoff mit R₅₋₁ in der zuvor angegebenen Bedeutung substituiert ist,
(cc) Furanyl, (dd) Thiophenyl,
wobei die Substituenten (a)-(dd) gegebenenfalls mit X und Y substituiert sind,
(ee) 1,2,3-, (ff) 1,2,4-Triazolyl, wobei der gesättigte Stickstoff mit R₅₋₁ in der zuvor angegebenen Bedeutung substituiert ist,
wobei die Substituenten (ee) und (ff) gegebenenfalls mit X substituiert sind;
(III) Y beim jeweiligen Auftreten unabhängig ausgewählt ist aus
(a) -H,
(b) -Fl, (c) -Cl, (d) -Br, (e) -I,
(f) -R₃₋₁, (g) -OR₃₋₁ mit R₃₋₁ gleich H oder C₁-C₄ Alkyl oder
(h) -NO₂;
(IV) X beim jeweiligen Auftreten unabhängig voneinander ausgewählt ist aus
(a) -H,
(b) C₁-C₈ Alkyl, gegebenenfalls substituiert durch ein oder mehrere Halogen(e),
-OH,
=O mit Ausnahme der α-Stellung,
-S(O)ₙR₃₋₂ mit R₃₋₂ gleich C₁-C₄ Alkyl oder C₃-C₈ Cycloalkyl oder
-NR₃₋₃R₃₋₄, wobei R₃₋₃ und R₃₋₄ gleich oder verschieden sein können und für -H, C₁-C₈ Alkyl, C₃-C₈ Cycloalkyl,
-(CH₂)ₜCHOR₃₋₅, -(CH₂)ₜNR₃₋₆R₃₋₇ stehen oder zusammen
-(CH₂)O(CH₂)- bilden,
-(CH₂)ₜCH(CO)R₃₋₈ oder -(CH₂)N(R₃₋₈)(CH₂)₂- mit
R₃₋₅ gleich -H oder C₁-C₄ Alkyl oder
R₃₋₆ und R₃₋₇ in gleicher oder verschiedener Bedeutung gleich -H, C₁-C₄ Alkyl oder zusammen gleich -(CH₂)ᵣ-,
(c) C₂-C₅ Alkenyl,
(d) C₃-C₈ Cycloalkyl,
(e) -OR₃₋₃ mit R₃₋₃ in der zuvor angegebenen Bedeutung,
(f) -CN,
(g) -S-(O)ₙ-R₃₋₈, mit R₃₋₈ gleich
C₁-C₄ Alkyl, gegebenenfalls substituiert durch ein oder mehrere Halogen(e), -OH, -CN, -NR₃₋₃R₃₋₄ mit R₃₋₃ und R₃₋₄ in der zuvor angegebenen Bedeutung, -CO₂R₃₋₅ mit R₃₋₅ in der zuvor angegebenen Bedeutung,
C₂-C₄ Alkenyl,
-NR₃₋₉R₃₋₁₀ mit R₃₋₉ gleich -H, C₁-C₄ Alkyl oder C₃-C₈ Cycloalkyl und R₃₋₁₀ gleich -H, C₁-C₄ Alkyl, C₁-C₄ Alkenyl, C₃-C₄ Cycloalkyl,
-OR₃₋₅ oder -NR₃₋₆R₃₋₇ mit R₃₋₅, R₃₋₆ und
R₃₋₇ in der zuvor angegebenen Bedeutung,
-N₃,
-NHC(O)R₃₋₁₁ mit R₃₋₁₁ gleich C₁-C₄ Alkyl, gegebenenfalls substituiert durch ein oder mehrere Halogen(e),
(h) -S(O)₂-N=S(O)ₚR₃₋₁₄R₃₋₁₅ mit R₃₋₁₄ und R₃₋₁₅ in gleicher oder verschiedener Bedeutung gleich C₁-C₂ Alkyl oder zusammen -(CH₂)_{q}-,
(i) -S-C(O)-R₃₋₁₁ mit R₃₋₁₁ in der zuvor angegebenen Bedeutung,
(j) Tetrazolyl,
(k) -NR₃₋₃R₃₋₄ mit R₃₋₃ und R₃₋₄ in der zuvor angegebenen Bedeutung,
(l) -N(R₃₋₃)COR₃₋₁₁ mit R₃₋₃ und R₃₋₁₁ in der zuvor angegebenen Bedeutung,
(m) -N(R₃₋₃)S(O)ₙR₃₋₁₁ mit R₃₋₃ und R₃₋₁₁ in der zuvor angegebenen Bedeutung,
(n) -CONR₃₋₃R₃₋₄ mit R₃₋₃ und R₃₋₄ in der zuvor angegebenen Bedeutung,
(o) -C(O)R₃₋₁₆ mit R₃₋₁₆ gleich
-H,
C₁-C₈ Alkyl, gegebenenfalls substituiert durch ein oder mehrere Halogen(e),
C₁-C₄ Alkyl, gegebenenfalls substituiert durch -OR₃₋₅, -OC(O)R₃₋₅, -NR₃₋₃R₃₋₄,
-S(O)ₙR₃₋₁₇,
C₃-C₈ Cycloalkyl oder
C₂-C₅ Alkenyl, gegebenenfalls substituiert durch -CHO oder -CO₂R₃₋₅, mit R₃₋₃, R₃₋₄ und
R₃₋₅ in der zuvor angegebenen Bedeutung und
R₃₋₁₇ gleich C₁-C₄ Alkyl oder C₃-C₈ Cycloalkyl,
(p) -C(=NR₃₋₁₈)R₃₋₁₆ mit R₃₋₁₆ in der zuvor angegebenen Bedeutung und R₃₋₁₈ gleich -NR₃₋₃R₃₋₄, -OR₃₋₃ oder -NHC(O)R₃₋₃ mit R₃₋₃ und R₃₋₄ in der zuvor angegebenen Bedeutung,
(q) -CR₃₋₁₆(OR₃₋₁₉)OR₃₋₂₀ mit R₃₋₁₆ in der zuvor angegebenen Bedeutung und R₃₋₁₉ und R₃₋₂₀ in gleicher oder verschiedener Bedeutung gleich C₁-C₄ Alkyl oder zusammen -(CH₂)m-, 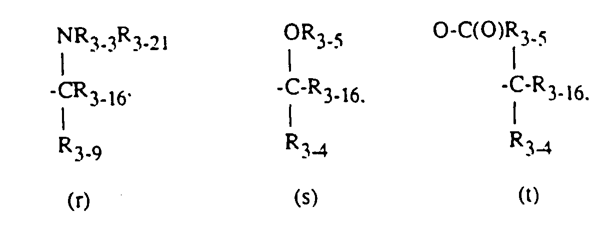 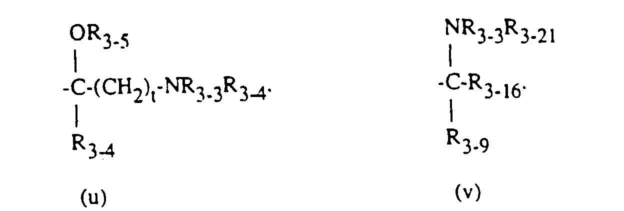 mit R₃₋₃, R₃₋₄, R₃₋₅, R₃₋₉ und R₃₋₁₆ in der zuvor angegebenen Bedeutung und R₃₋₂₁ gleich R₃₋₄ oder -NR₃₋₄R₃₋₅ mit R₃₋₄ und R₃₋₅ in der zuvor angegebenen Bedeutung,
m = 2 oder 3,
n = 0, 1 oder 2,
p = 0 oder 1,
q = 3, 4 oder 5,
t = 1, 2 oder 3;
(V) R₄ ausgewählt ist aus
(a) -H,
(b) C₁-C₁₂ Alkyl, gegebenenfalls substituiert mit 1-3 Cl,
(c) C₃-C₁₂ Cycloalkyl,
(d) C₅-C₁₂ Alkenyl mit einer Doppelbindung,
(e) Phenyl, gegebenenfalls substituiert durch 1-3 -OH, -OCH₃, -OC₂H₅, -NO₂, -F, -Cl, -Br, -COOH und -SO₃H, -N(R₄₋₁)(R₄₋₂) mit R₄₋₁ und R₄₋₂ in gleicher oder verschiedener Bedeutung gleich -H oder C₁-C₅ Alkyl,
(f) Furanyl,
(g) Tetrahydrofuranyl,
(h) 2-Thiophen,
(i) Pyrrolidinyl,
(j) Pyridinyl,
(k) -O-R₄₋₃ mit R₄₋₃ gleich C₁-C₄ Alkyl,
(l) -NH₂,
(m) -NHR₄₋₄ mit R₄₋₄ gleich C₁-C₃ Alkyl oder Phenyl,
(n) -NR₄₋₄R₄₋₅ mit R₄₋₄ in der zuvor angegebenen Bedeutung und R₄₋₅ gleich C₁-C₃ Alkyl oder R₄₋₄ und R₄₋₅ zusammen mit dem daran hängenden Stickstoffatom gleich einem gesättigten, ein Stickstoffatom enthaltenden heterocyclischen C₅-C₇-0Ring der -O- umfaßt (Morpholin),
(o) -CH₂-OH oder
(p) -CH₂-OR₄₋₆ mit R₄₋₆ gleich C₁-C₄ Alkyl oder -CO-R₄₋₇ mit R₄₋₇ gleich C₁-C₄ Alkyl oder Phenyl,
oder ein pharmazeutisch akzeptables Salz desselben.

2. Verbindung nach Anspruch 1, wobei R₁ für H oder F steht.

3. Verbindung nach Anspruch 2, wobei R₃ für gegebenenfalls mit X und Y substituiertes Phenyl steht.

4. Verbindung nach Anspruch 3, nämlich
(±)-5-(Acetamidomethyl)-3-(4-phenyl-3-fluorphenyl)-2-oxazolidinon,
(S)-N-[[3-[3-Fluor-4-(4-pyridyl)-phenyl]-2-oxo-5-oxazolidinyl]-methyl]-acetamid,
(±)-5-(Acetamidomethyl)-3-[4-(4-(dimethylamino)phenyl)-3-fluorphenyl]-2-oxazolidinon,
(±)-5-(Acetamidomethyl)-3-(4-phenyl-3,5-fluorphenyl)-2-oxazolidinon oder
(±)-5-(Acetamidomethyl)-3-[4-(4-(dimethylamino)phenyl)-3,5-difluorphenyl]-2-oxazolidinon.

5. Verbindung nach Anspruch 2, wobei R₃ für gegebenenfalls durch X und Y substituiertes Pyridyl steht.

6. Verbindung nach Anspruch 5, nämlich
(±)-5-(Acetamidomethyl)-3-[4-(3-pyridyl)-3-fluorphenyl]-2-oxazolidinon,
(±)-5-(Acetamidomethyl)-3-[4-(4-pyridyl)-3-fluorphenyl]-2-oxazolidinon,
(±)-5-(Acetamidomethyl)-3-[4-(2,6-dimethylpyridin-4-yl)-3-fluorphenyl]-2-oxazolidinon,
(±)-5-(Acetamidomethyl)-3-[4-(2-methylpyridin-4-yl)-3-fluorphenyl]-2-oxazolidinon,
(±)-5-(Acetamidomethyl)-3-[4-(2-ethylpyridin-4-yl)-3-fluorphenyl]-2-oxazolidinon,
(±)-5-(Acetamidomethyl)-3-[4-(3-pyridyl)-3,5-difluorphenyl]-2-oxazolidinon,
(±)-5-(Acetamidomethyl)-3-[4-(3-pyridyl)-3,5-difluorphenyl]-2-oxazolidinon-ethansulfonsäuresalz,
(±)-5-(Acetamidomethyl)-3-[4-(4-pyridyl)-3,5-difluorphenyl]-2-oxazolidinon,
(±)-5-(Acetamidomethyl)-3-[4-(2,6-dimethylpyridin-4-yl)-3,5-difluorphenyl]-2-oxazolidinon,
(±)-5-(Acetamidomethyl)-3-[4-(2-methylpyridin-4-yl)-3,5-difluorphenyl]-2-oxazolidinon oder
(±)-5-(Acetamidomethyl)-3-[4-(2-ethylpyridin-4-yl)-3,5-difluorphenyl]-2-oxazolidinon.

7. Verbindung nach Anspruch 2, wobei R₃ für gegebenenfalls durch X und Y substituiertes Chinolinyl oder Isochinolinyl steht.

8. Verbindung nach Anspruch 7, nämlich
(±)-5-(Acetamidomethyl)-3-[4-(3-chinolyl)-3-fluorphenyl]-2-oxazolidinon,
(±)-5-(Acetamidomethyl)-3-[4-(4-chinolyl)-3-fluorphenyl]-2-oxazolidinon,
(±)-5-(Acetamidomethyl)-3-[4-(6-chinolyl)-3-fluorphenyl]-2-oxazolidinon,
(±)-5-(Acetamidomethyl)-3-[4-(4-isochinolyl)-3-fluorphenyl]-2-oxazolidinon,
(±)-5-(Acetamidomethyl)-3-[4-(3-chinolyl)-3,5-difluorphenyl]-2-oxazolidinon,
(±)-5-(Acetamidomethyl)-3-[4-(4-chinolyl)-3,5-difluorphenyl]-2-oxazolidinon,
(±)-5-(Acetamidomethyl)-3-[4-(6-chinolyl)-3,5-difluorphenyl]-2-oxazolidinon oder
(±)-5-(Acetamidomethyl)-3-[4-(4-isochinolyl)-3,5-difluorphenyl]-2-oxazolidinon.

9. Verbindung nach Anspruch 2, wobei R₃ für gegebenenfalls durch X und Y substituiertes Indolyl mit gegebenenfalls durch R₅ substituiertem Stickstoff steht.

10. Verbindung nach Anspruch 9, nämlich
(±)-5-(Acetamidomethyl)-3-[4-(5-indolyl)-3-fluorphenyl]-2-oxazolidinon,
(±)-5-(Acetamidomethyl)-3-[4-(1-methyl-5-indolyl)-3-fluorphenyl]-2-oxazolidinon,
(±)-5-(Acetamidomethyl)-3-[4-(5-indolyli-3,5-difluorphenyl]-2-oxazolidinon oder
(±)-5-(Acetamidomethyl)-3-[4-(1-methyl-5-indolyl)-3,5-difluorphenyl]-2-oxazolidinon.

11. Verbindung nach Anspruch 2, wobei R₃ für gegebenenfalls mit X und Y substituiertes Benzothiazolyl oder Benzoxazolyl steht.

12. Verbindung nach Anspruch 11, nämlich
(±)-5-(Acetamidomethyl)-3-[4-(6-benzothiazolyl)-3-fluorphenyl]-2-oxazolidinon,
(±)-5-(Acetamidomethyl)-3-[4-(6-benzoxazolyl)-3-fluorphenyl]-2-oxazolidinon,
(±)-5-(Acetamidomethyl)-3-[4-(6-benzothiazolyl)-3,5-difluorphenyl]-2-oxazolidinon oder
(±)-5-(Acetamidomethyl)-3-[4-(6-benzoxazolyl)-3,5-difluorphenyl]-2-oxazolidinon.

13. Verbindung nach Anspruch 2, wobei R₃ für gegebenenfalls mit X und Y substituiertes Thiazolyl oder Oxazolyl steht.

14. Verbindung nach Anspruch 13, nämlich
(±)-5-(Acetamidomethyl)-3-[4-(2-amino-4-thiazolyl)-3-fluorphenyl]-2-oxazolidinon,
(±)-5-(Acetamidomethyl)-3-[4-(2-amino-4-oxazolyl)-3-fluorphenyl]-2-oxazolidinon,
(±)-5-(Acetamidomethyl)-3-[4-(2-dimethylamino)-4-thiazolyl)-3,5-difluorphenyl]-2-oxazolidinon,
(±)-5-(Acetamidomethyl)-3-[4-(2-amino-4-thiazolyl)-3,5-difluorphenyl]-2-oxazolidinon,
(±)-5-(Acetamidomethyl)-3-[4-(2-dimethylamino)-4-oxazolyl)-3,5-difluorphenyl]-2-oxazolidinon oder
(±)-5-(Acetamidomethyl)-3-[4-(2-amino-4-oxazolyl)-3,5-difluorphenyl]-2-oxazolidinon.

15. Verbindung nach einem der vorhergehenden Ansprüche, worin R₁ für F steht.

16. Verfahren zur Herstellung einer Verbindung der Formel (XII) gemäß der Definition von Anspruch 1 durch
(a) Umwandeln eines substituierten Anilins in ein Stabasederivat;
(b) Behandeln des Stabasederivats zur Bildung eines aryl- oder heteroarylsubstituierten Anilins und
(c) Umwandeln des aryl- oder heteroarylsubstituierten Anilins in ein aryl- oder heteroarylsubstituiertes Phenyloxazolidinon.

17. Verfahren zur Herstellung eines Oxazolidinonjodids durch Umsetzen einer Carbobenzyloxyallylverbindung in Gegenwart von überschüssigem Pyridin und Jod, wobei der Überschuß aus gleichen Mengen vonn 2 - 20 Moläquivalenten besteht.

## Revendications

1. Aryl- et hétéroaryl-phényl-oxazolidinones substituées, de formule (XII) 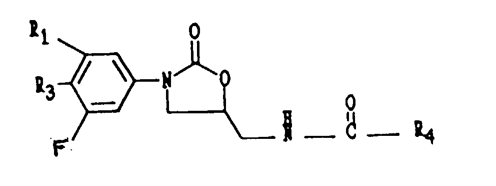 dans laquelle
(I) R₁ est choisi entre
(a) -H,
(b) -F,
(c) -Cl,
(d) un groupe -CF₃, et
(f) un groupe -OCH₃ ;
(II) R₃ est choisi dans le groupe consistant en
(a) un groupe phényle,
(b) un groupe pyridyle,
(c) un groupe pyrazinyle, (d) un groupe pyridazinyle, (e) un groupe pyrimidinyle,
(f) un groupe 1,2,3-triazinyle, (g) un groupe 1,2,4-triazinyle, (h) un groupe 1,2,5-triazinyle,
(i) un groupe quinolinyle, (j) un groupe isoquinolinyle,
(k) un groupe quinoxalinyle, (l) un groupe quinazolinyle, (m) un groupe phtalazinyle, (n) un groupe cinnolinyle,
(o) un groupe naphtyridinyle,
(p) un groupe indolyle ayant l'atome d'azote facultativement substitué avec un groupe R₅₋₁ dans lequel R₅₋₁ représente
-H,
un groupe alkyle en C₁ à C₄ facultativement substitué avec un ou plusieurs halogènes,
un groupe cycloalkyle en C₃ à C₆, ou
un groupe C(O)R₅₋₂ dans lequel R₅₋₂ représente
-H,
un groupe alkyle en C₁ à C₄ facultativement substitué avec un ou plusieurs halogènes
un groupe phényle facultativement substitué avec un ou plusieurs halogènes,
(q) un groupe pyrrolopyridinyle ayant l'atome d'azote saturé substitué avec un groupe R₅₋₁, le groupe R₅₋₁ répondant à la définition précitée, (r) un groupe furannopyridinyle, (s) un groupe thiénopyridinyle,
(t) un groupe benzothiazolyle, (u) un groupe benzoxazolyle,
(v) un groupe imidazolyle ayant l'atome d'azote saturé substitué avec un groupe R₅₋₁, le groupe R₅₋₁ répondant à la définition précitée,
(w) un groupe pyrazolyle ayant l'atome d'azote saturé substitué avec un groupe R₅₋₁, le groupe R₅₋₁ répondant à la définition précitée,
(x) un groupe thiazolyle, (y) un groupe isothiazolyle,
(z) un groupe oxazolyle, (aa) un groupe isoxazolyle,
(bb) un groupe pyrroyle ayant l'atome d'azote substitué avec un groupe R₅₋₁, le groupe R₅₋₁ répondant à la définition précitée,
(cc) un groupe furannyle, (dd) un groupe thiophényle,
les substituants (a) à (dd) étant facultativement substitués avec des groupes X et Y,
(ee) un groupe 1,2,3-triazolyle, (ff) un groupe 1,2,4-triazolyle ayant l'atome d'azote saturé substitué avec un groupe R₅₋₁, le groupe R₅₋₁ répondant à la définition précitée,
les substituants (ee) et (ff) étant facultativement substitués avec X ;
(III) chaque groupe Y présent est choisi indépendamment entre
(a) -H,
(b) -Fl, (c) -Cl, (d) -Br, (e) -I,
(f) R₃₋₁, (g) -OR₃₋₁ dans lequel R₃₋₁ représente H ou un groupe alkyle en C₁ à C₄, ou
(h) -NO₂ ;
(IV) chaque groupe X présent est choisi indépendamment entre
(a) -H,
(b) un groupe alkyle en C₁ à C₈ facultativement substitué avec
un ou plusieurs halogènes,
un groupe -OH,
un groupe =O autre qu'un groupe en position alpha,
un groupe -S(O)ₙR₃₋₂ dans lequel R₃₋₂ représente un groupe alkyle en C₁ à C₄ ou cycloalkyle en C₃ à C₈, ou
un groupe -NR₃₋₃R₃₋₄ dans lequel R₃₋₃ et R₃₋₄ sont identiques ou différents et représentent -H, un groupe alkyle en C₁ à C₈, cycloalkyle en C₃ à C₈, -(CH₂)ₜCHOR₃₋₅, -(CH₂)ₜNR₃₋₆R₃₋₇, ou bien, pris conjointement, représentent un groupe (CH₂)O(CH₂)-, -(CH₂)ₜCH(CO)R₃₋₈ ou -(CH₂)N(R₃₋₈)(CH₂)₂-, groupes dans lesquels
R₃₋₅ représente H ou un groupe alkyle en C₁ à C₄, ou
R₃₋₆ et R₃₋₇ sont identiques ou différents et représentent -H ou un groupe alkyle en C₁ à C₄ ou bien, pris conjontement, représentent un groupe -(CH₂)ᵣ-,
(c) un groupe alcényle en C₂ à C₅,
(d) un groupe cycloalkyle en C₃ à C₈,
(e) un groupe -OR₃₋₃ dans lequel R₃₋₃ répond à la définition précitée,
(f) un groupe -CN,
(g) un groupe -S-(O)ₙ-R₃₋₈ dans lequel R₃₋₈ représente
un groupe alkyle en C₁ à C₄ facultativement substitué avec
un ou plusieurs halogènes,
un groupe -OH,
un groupe -CN,
un groupe NR₃₋₃R₃₋₄ dans lequel R₃₋₃ et R₃₋₄ répondent aux définitions précitées,
un groupe -CO₂R₃₋₅ dans lequel R₃₋₅ répond à la définition précitée,
un groupe alcényle en C₂ à C₄,
un groupe -NR₃₋₉R₃₋₁₀ dans lequel R₃₋₉ représente H, un groupe alkyle en C₁ à C₄, cycloalkyle en C₃ à C₈ et R₃₋₁₀ représente -H, un groupe alkyle en C₁ à C₄, alcényle en C₁ à C₄, cycloalkyle en C₃ ou C₄, -OR₃₋₅ ou -NR₃₋₆R₃₋₇ dans lequel R₃₋₅, R₃₋₆ et R₃₋₇ répondent aux définitions précitées,
-N₃,
un groupe -NHC(O)R₃₋₁₁ dans lequel R₃₋₁₁ représente un groupe alkyle en C₁ à C₄ facultativement substitué avec un ou plusieurs halogènes,
(h) un groupe -S(O)₂-N=S(O)ₚR₃₋₁₄R₃₋₁₅ dans lequel R₃₋₁₄ et R₃₋₁₅ sont identiques ou différents et représentent un groupe alkyle en C₁ à C₄ ou, pris conjointement, représentent -(CH₂)_{q}-,
(i) un groupe -S-C(O)-R₃₋₁₁ dans lequel R₃₋₁₁ répond à la définition précitée,
(j) un groupe tétrazolyle,
(k) un groupe NR₃₋₃R₃₋₄ dans lequel R₃₋₃ et R₃₋₄ répondent aux définitions précitées,
(l) un groupe -N(R₃₋₃)COR₃₋₁₁ dans lequel R₃₋₃ et R₃₋₁₁ répondent aux définitions précitées,
(m) un groupe -N(R₃₋₃)S(O)ₙR₃₋₁₁ dans lequel R₃₋₃ et R₃₋₁₁ répondent aux définitions précitées,
(n) un groupe -CONR₃₋₃R₃₋₄ dans lequel R₃₋₃ et R₃₋₄ répondent aux définitions précitées,
(o) un groupe -C(O)R₃₋₁₆ dans lequel R₃₋₁₆ représente
-H,
un groupe alkyle en C₁ à C₈ facultativement substitué avec un ou plusieurs halogènes,
un groupe alkyle en C₁ à C₄ facultativement substitué avec
un groupe -OR₃₋₅,
un groupe -OC(O)R₃₋₅,
un groupe -NR₃₋₃R₃₋₄,
un groupe -S(O)ₙR₃₋₁₇,
un groupe cycloalkyle en C₃ à C₈, ou
un groupe alcényle en C₂ à C₅ facultativement substitué avec un groupe -CHO ou -CO₂R₃₋₅, groupes dans lesquels R₃₋₃, R₃₋₄ et R₃₋₅ répondent aux définitions précitées et R₃₋₁₇ représente un groupe alkyle en C₁ à C₄ ou cycloalkyle en C₃ à C₈,
(p) un groupe -C(=NR₃₋₁₈)R₃₋₁₆ dans lequel R₃₋₁₆ répond à la définition précitée et R₃₋₁₈ représente un groupe -NR₃₋₃R₃₋₄, -OR₃₋₃ ou -NHC(O)R₃₋₃ dans lequel R₃₋₃ et R₃₋₄ répondent aux définitions précitées,
(q) un groupe -CR₃₋₁₆(OR₃₋₁₉)OR₃₋₂₀ dans lequel R₃₋₁₆ répond à la définition précitée et R₃₋₁₉ et R₃₋₂₀ sont identiques ou différents et représentent un groupe alkyle en C₁ à C₄ ou bien, pris conjointement, représentent un groupe -(CH₂)ₘ-, 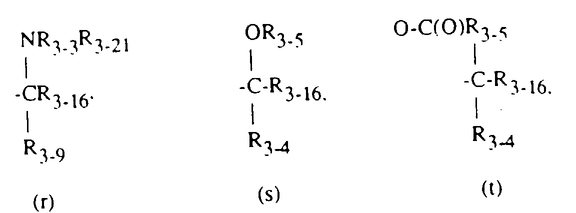 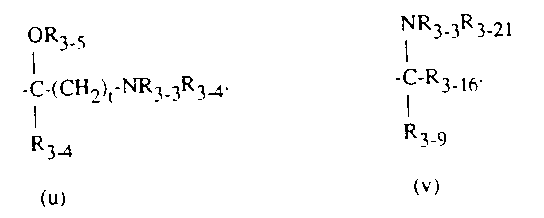 formules dans lesquelles R₃₋₃, R₃₋₄, R₃₋₅, R₃₋₉ et R₃₋₁₆ répondent aux définitions précitées et R₃₋₂₁ représente un groupe R₃₋₄ ou -NR₃₋₄R₃₋₅ dans lequel R₃₋₄ et R₃₋₅ répondent aux définitions précitées,
m est égal à 2 ou 3 ;
n est égal à 0, 1 ou 2 ;
p est égal à 0 ou 1 ;
q est égal à 3, 4 ou 5 ;
t est égal à 1, 2 ou 3 ;
(V) R₄ est choisi dans le groupe consistant en
(a) -H,
(b) Un groupe alkyle en C₁ à C₁₂ facultativement substitué avec 1 à 3 groupes Cl,
(c) un groupe cycloalkyle en C₃ à C₁₂,
(d) un groupe alcényle en C₅ à C₁₂ contenant une double liaison,
(e) un groupe phényle facultativement substitué avec 1 à 3 des groupes consistant en -OH, -OCH₃, -OC₂H₅, -NO₂, F, -Cl, -Br, -COOH et -SO₃H, -N(R₄₋₁)(R₄₋₂) dans lequel R₄₋₁ et R₄₋₂ sont identiques ou différents et représentent -H ou un groupe alkyle en C₁ à C₅,
(f) un groupe furannyle,
(g) un groupe tétrahydrofurannyle,
(h) un groupe 2-thiophène,
(i) un groupe pyrrolidinyle,
(j) un groupe pyridinyle,
(k) un groupe -O-R₄₋₃ dans lequel R₄₋₃ représente un groupe alkyle en C₁ à C₄,
(l) un groupe -NH₂,
(m) un groupe -NHR₄₋₄ dans lequel R₄₋₄ représente un groupe alkyle en C₁ à C₃ ou un groupe φ,
(n) un groupe -NR₄₋₄R₄₋₅ dans lequel R₄₋₄ répond à la définition précitée et R₄₋₅ représente un groupe alkyle en C₁ à C₃, ou
R₄₋₄ et R₄₋₅ sont pris conjointement avec l'atome d'azote auquel ils sont fixés en formant un noyau hétérocyclique en C₅ à C₇ mono-azoté saturé comprenant le groupe -O- (morpholine),
(o) un groupe -CH₂-OH, ou
(p) un groupe -CH₂-OR₄₋₆ dans lequel R₄₋₆ représente un groupe alkyle en C₁ à C₄ ou -CO-R₄₋₇ dans lequel R₄₋₇ représente un groupe alkyle en C₁ à C₄ ou un groupe -φ ;
ou leurs sels pharmaceutiquement acceptables.

2. Composé suivant la revendication 1, dans lequel R₁ représente H ou F.

3. Composé suivant la revendication 2, dans lequel R₃ représente un groupe phényle facultativement substitué avec X et Y.

4. Composé suivant la revendication 1, qui consiste en l'un des suivants :
(±)-5-(acétamidométhyl)-3-(4-phényl-3-fluorophényl)-2-oxazolidinone,
(S)-N-[[3-[3-fluoro-4-(4-pyridyl)phényl]-2-oxo-5-oxazolidinyl]méthyl]acétamide,
(±)-5-(acétamidométhyl)-3-[4-(4-(diméthylamino)phényl)-3-fluorophényl]-2-oxazolidinone,
(±)-5-(acétamidométhyl)-3-(4-phényl-3,5-fluorophényl)-2-oxazolidinone,
(±)-5-(acétamidométhyl)-3-[4-[4-(diméthylamino)phényl)-3,5-difluorophényl]-2-oxazolidinone.

5. Composé suivant la revendication 2, dans lequel R₃ représente un groupe pyridyle facultativement substitué avec X et Y.

6. Composé suivant la revendication 5, qui consiste en l'un des suivants :
(±)-5-(acétamidométhyl)-3-[4-(3-pyridyl)-3-fluorophényl]-2-oxazolidinone,
(±)-5-(acétamidométhyl)-3-[4-(4-pyridyl)-3-fluorophényl]-2-oxazolidinone,
(±)-5-(acétamidométhyl)-3-[4-(2,6-diméthylpyridine-4-yl)-3-fluorophényl]-2-oxazolidinone,
(±)-5-(acétamidométhyl)-3-[4-(2-méthylpyridine-4-yl)-3-fluorophényl]-2-oxazolidinone,
(±)-5-(acétamidométhyl)-3-[4-(2-éthylpyridine-4-yl)-3-fluorophényl]-2-oxazolidinone,
(±)-5-(acétamidométhyl)-3-[4-(3-pyridyl)-3,5-difluorophényl]-2-oxazolidinone,
(±) -5-(acétamidométhyl)-3-[4-(3-pyridyl)-3,5-difluorophényl]-2-oxazolidinone, sel méthanesulfonique,
(±)-5-(acétamidométhyl)-3-[4-(4-pyridyl)-3,5-difluorophényl]-2-oxazolidinone,
(±)-5-(acétamidométhyl)-3-[4-(2,6-diméthylpyridine-4-yl)-3,5-difluorophényl]-2-oxazolidinone,
(±)-5-(acétamidométhyl)-3-[4-(2-méthylpyridine-4-yl)-3,5-difluorophényl]-2-oxazolidinone,
(±)-5-(acétamidométhyl)-3-[4-(2-éthylpyridine-4-yl)-3,5-difluorophényl]-2-oxazolidinone.

7. Composé suivant la revendication 2, dans lequel R₃ représente un groupe quinolinyle ou isoquinolinyle facultativement substitué avec X et Y.

8. Composé suivant la revendication 7, qui consiste en l'un des suivants :
(±)-5-(acétamidométhyl)-3-[4-(3-quinolyl)-3-fluorophényl]-2-oxazolidinone,
(±)-5-(acétamidométhyl)-3-[4-(4-quinolyl)-3-fluorophényl]-2-oxazolidinone,
(±)-5-(acétamidométhyl)-3-[4-(6-quinolyl)-3-fluorophényl]-2-oxazolidinone,
(±)-5-(acétamidométhyl)-3-[4-(4-isoquinolyl)-3-fluorophényl]-2-oxazolidinone,
(±)-5-(acétamidométhyl)-3-[4-(3-quinolyl)-3,5-difluorophényl]-2-oxazolidinone,
(±)-5-(acétamidométhyl)-3-[4-(4-quinolyl)-3,5-difluorophényl]-2-oxazolidinone,
(±)-5-(acétamidométhyl)-3-[4-(6-quinolyl)-3,5-difluorophényl]-2-oxazolidinone,
(±)-5-(acétamidométhyl)-3-[4-(4-isoquinolyl)-3,5-difluorophényl]-2-oxazolidinone.

9. Composé suivant la revendication 2, dans lequel R₃ représente un groupe indolyle ayant un atome d'azote facultativement substitué avec un groupe R₅ et facultativement substitué avec X et Y.

10. Composé suivant la revendication 9, qui consiste en l'un des suivants :
(±)-5-(acétamidométhyl)-3-[4-(5-indolyl)-3-fluorophényl]-2-oxazolidinone,
(±)-5-(acétamidométhyl)-3-[4-(1-méthyl-5-indolyl)-3-fluorophényl]-2-oxazolidinone,
(±)-5-(acétamidométhyl)-3-[4-(5-indolyl)-3,5-difluorophényl]-2-oxazolidinone,
(±)-5-(acétamidométhyl)-3-[4-(1-méthyl-5-indolyl)-3,5-difluorophényl]-2-oxazolidinone.

11. Composé suivant la revendication 2, dans lequel R₃ représente un groupe benzothiazolyle ou benzoxazolyle facultativement substitué avec X et Y.

12. Composé suivant la revendication 11, qui consiste en l'un des suivants :
(±)-5-(acétamidométhyl)-3-[4-(6-benzothiazolyl)-3-fluorophényl]-2-oxazolidinone,
(±)-5-(acétamidométhyl)-3-[4-(6-benzoxazolyl)-3-fluorophényl]-2-oxazolidinone,
(±)-5-(acétamidométhyl)-3-[4-(6-benzothiazolyl)-3,5-difluorophényl]-2-oxazolidinone,
(±)-5-(acétamidométhyl)-3-[4-(6-benzoxazolyl)-3,5-difluorophényl]-2-oxazolidinone.

13. Composé suivant la revendication 2, dans lequel R₃ représente un groupe thiazolyle ou oxazolyle facultativement substitué avec X et Y.

14. Composé suivant la revendication 13, qui consiste en l'un des suivants :
(±)-5-(acétamidométhyl)-3-[4-(2-amino-4-thiazolyl)-3-fluorophényl]-2-oxazolidinone,
(±)-5-(acétamidométhyl)-3-[4-(2-amino-4-oxazolyl)-3-fluorophényl]-2-oxazolidinone,
(±)-5-(acétamidométhyl)-3-[4-(2-diméthylamino-4-thiazolyl)-3,5-difluorophényl]-2-oxazolidinone,
(±)-5-(acétamidométhyl)-3-[4-(2-amino-4-thiazolyl)-3,5-difluorophényl]-2-oxazolidinone,
(±)-5-(acétamidométhyl)-3-[4-(2-diméthylamino-4-oxazolyl)-3,5-difluorophényl]-2-oxazolidinone,
(±)-5-(acétamidométhyl)-3-[4-(2-amino-4-oxazolyl)-3,5-difluorophényl]-2-oxazolidinone.

15. Composé suivant l'une quelconque des revendications précédentes, dans lequel R₁ représente F.

16. Procédé de préparation d'un composé de formule (XII), répondant à la définition suivant la revendication 1, comprenant les étapes consistant :
(a) à transformer une aniline substituée en un dérivé de stabase ;
(b) à traiter le dérivé de stabase pour former une aniline à subtituant aryle ou hétéroaryle ; et
(c) à transformer l'aniline à substituant aryle ou hétéroaryle en une phényloxazolidinone à substituant aryle ou hétéroaryle.

17. Procédé de préparation d'un iodure d'oxazolidinone, comprenant la réaction d'un composé de carbobenzyloxyallyle en présence d'un excès de pyridine et d'iode, l'excès étant égal à des quantités de 2 à 20 équivalents molaires.
